# EUROPEAN PATENT SPECIFICATION

(11) **EP 3 381 350 B1**
(45) Date of publication and mention of the grant of the patent: **09.02.2022**
(21) Application number: 18164514.4
(22) Date of filing: 28.03.2018
(51) Int. Cl.: A61B 3/02, A61B 3/032

(54) **SUBJECTIVE OPTOMETRY APPARATUS AND SUBJECTIVE OPTOMETRY PROGRAM**
VORRICHTUNG ZUR SUBJEKTIVEN OPTOMETRIE UND PROGRAMM ZUR SUBJEKTIVEN OPTOMETRIE
APPAREIL D'OPTOMÉTRIE SUBJECTIVE ET PROGRAMME D'OPTOMÉTRIE SUBJECTIVE

(30) Priority: 31.03.2017 JP 2017070652; 31.03.2017 JP 2017070653
(43) Date of publication of application: 03.10.2018
(73) Proprietor: Nidek Co., Ltd., Gamagori-shi, Aichi, 443-0038 (JP)
(72) Inventor: TAKII, Michihiro, Gamagori, Aichi (JP); HANEBUCHI, Masaaki, Gamagori, Aichi (JP)
(74) Representative: Hoefer & Partner Patentanwälte mbB

(56) References cited:
- EP-A1- 2 899 585
- WO-A1-2016/149536
- JP-B2- 5 375 201

## Description

### TECHNICAL FIELD

The present disclosure relates to a subjective optometry apparatus and a subjective optometry program for measuring optical characteristics of a examinee's eye.

### BACKGROUND

A subjective optometry apparatus which measures optical characteristics (refractive power or the like) of a examinee's eye by disposing an optical element, such as a spherical lens or a cylindrical surface lens, in front of the eyes of an examinee and by presenting an examination visual target to the examinee's eye via the optical element, is known (for example, refer to JP-A-H05-176893 or EP 2 899 585 A1).

In subjective examination, the examinee's eye and the subjective optometry apparatus are aligned and the examination visual target that corresponds to an eye refractive power of the examinee's eye is presented to the examinee's eye. However, in a case were the target light flux is incident on the optical element from an off-axis position, distortion may occur in the examination visual target guided to the examinee's eye. In this state, since the examination visual target deformed by the distortion is guided to the examinee's eye, it is difficult to correctly measure the optical characteristics of the examinee's eye. In addition, in a case where the target light flux is incident on the optical element from the off-axis position, a light condensing position of the target light flux with respect to the examinee's eye is shifted in a center region and a peripheral region of the examination visual target. In this state, an image plane of an image of the examination visual target guided to the examinee's eye is tilted, and it is difficult to correctly measure the optical characteristics of the examinee's eye.

### SUMMARY

An object of the present disclosure is to provide a subjective optometry apparatus and a subjective optometry program which can easily perform subjective examination with high accuracy.

In order to solve the above-described problem, the present disclosure includes the following configurations.
(1) A subjective optometry apparatus for subjectively measuring optical characteristics of an examinee's eye, including:
   a light projecting optical system that projects a target light flux to the examinee's eye;
   a calibration optical system that is disposed in an optical path from the light projecting optical system to the examinee's eye, and changes optical characteristics of the target light flux;
   an optical element that guides the target light flux of which the optical characteristics is changed by the calibration optical system to the examinee's eye; and
   a correction portion that corrects distortion of the target light flux caused by the target light flux passing through an optical path deviated from an optical axis of the optical element,
   in which the target light flux passes through the optical path deviated from the optical axis of the optical element and is guided to the examinee's eye, and
   the subjective optometry apparatus subjectively measures the optical characteristics of the examinee's eye using the target light flux guided to the examinee's eye.
(2) The subjective optometry apparatus according to the above-described (1), in which the correction portion corrects the distortion of the target light flux based on a calibration power of the calibration optical system.
(3) The subjective optometry apparatus according to the above-described (1) or (2), further including:
   a measurement unit that accommodates the light projecting optical system; and
   a shift detection portion that detects a positional shift of the target light flux with respect to the examinee's eye,
   in which the correction portion corrects the distortion of the target light flux based on a detection result detected by the shift detection portion.
(4) The subjective optometry apparatus according to any one of the above-described (1) to (3), in which the light projecting optical system includes a display, and the target light flux is emitted as a visual target is displayed on the display, and
   the correction portion deforms the visual target displayed on the display to correct the distortion of the target light flux.
(5) The subjective optometry apparatus according to the above-described (4), in which the correction portion deforms the visual target to correct the distortion of the target light flux by performing at least one processing among a change in size in a longitudinal direction, a change in size in a lateral direction, and a movement of the visual target, in the visual target displayed on the display.
(6) The subjective optometry apparatus according to any one of the above-described (1) to (3), further including:
   a moving optical element that is movable with respect to an optical path of the light projecting optical system; and
   a driving portion that moves the moving optical element with respect to the optical path of the light projecting optical system,
   in which the correction portion that controls the driving portion for moving the moving optical element to correct the distortion of the target light flux.
(7) The subjective optometry apparatus according to any one of the above-described (1) to (5), in which the optical element guides the target light flux to the examinee's eye so as to have an optically predetermined examination distance.
(8) A subjective optometry apparatus for subjectively measuring optical characteristics of an examinee's eye, including:
   a light projecting optical system that projects a target light flux to the examinee's eye;
   a calibration optical system that is disposed in an optical path from the light projecting optical system to the examinee's eye, and changes optical characteristics of the target light flux;
   an optical element that guides the target light flux of which the optical characteristics is changed by the calibration optical system to the examinee's eye; and
   a correction portion that corrects aberration caused by the target light flux passing through an optical path deviated from an optical axis of the optical element, based on a calibration power of the calibration optical system,
   in which the target light flux passes through the optical path deviated from the optical axis of the optical element to be guided to the examinee's eye, and
   the subjective optometry apparatus subjectively measures the optical characteristics of the examinee's eye using the target light flux guided to the examinee's eye.
(9) The subjective optometry apparatus according to the above-described (1) or (8), in which the correction portion corrects inclination of an image plane of an image of the target light flux caused by the target light flux passing through the optical path deviated from the optical axis of the optical element.
(10) The subjective optometry apparatus according to the above-described (9), in which the correction portion corrects the inclination of the image plane of the image of the target light flux based on a calibration power of the calibration optical system.
(11) The subjective optometry apparatus according to the above-described (9) or (10), further including:
   a measurement unit that accommodates the light projecting optical system; and
   a shift detection portion that detects a positional shift of the target light flux with respect to the examinee's eye,
   in which the correction portion corrects the inclination of the image plane of the image of the target light flux based on a detection result detected by the shift detection portion.
(12) The subjective optometry apparatus according to any one of the above-described (9) to (11), in which the light projecting optical system includes a display, and the target light flux is emitted as a visual target is displayed on the display, and
   the correction portion changes an angle of a surface of the display with respect to the optical axis to correct the inclination of the image plane of the image of the target light flux.
(13) The subjective optometry apparatus according to any one of the above-described (9) to (11), further including:
   a moving optical element that is movable with respect to an optical path of the light projecting optical system; and
   a driving portion that moves the moving optical element with respect to the optical path of the light projecting optical system,
   in which the correction portion controls the driving portion for moving the moving optical element to correct the inclination of the image plane of the image of the target light flux.
(14) The subjective optometry apparatus according to any one of the above-described (9) to (13), in which the optical element guides an image of the target light flux to the examinee's eye so as to have an optically predetermined examination distance.
(15) A subjective optometry program used in a subjective optometry apparatus including a light projecting optical system that projects a target light flux to an examinee's eye, a calibration optical system that is disposed in an optical path from the light projecting optical system to the examinee's eye, and changes optical characteristics of the target light flux, and an optical element that guides the target light flux of which the optical characteristics is changed by the calibration optical system to the examinee's eye, in which the target light flux passes through the optical path deviated from an optical axis of the optical element to be guided to the examinee's eye, and optical characteristics of the examinee's eye are subjectively measured using the target light flux guided to the examinee's eye,
   the subjective optometry program, when executed by a processor of the subjective optometry apparatus, causing the subjective optometry apparatus to perform a correction step of correcting distortion of the target light flux caused by the target light flux passing through an optical path deviated from an optical axis of the optical element.

(16) A subjective optometry program used in a subjective optometry apparatus including a light projecting optical system that projects a target light flux to an examinee's eye, a calibration optical system that is disposed in an optical path from the light projecting optical system to the examinee's eye, and changes optical characteristics of the target light flux, and an optical element that guides the target light flux of which the optical characteristics is changed by the calibration optical system to the examinee's eye, in which the target light flux passes through the optical path deviated from an optical axis of the optical element to be guided to the examinee's eye, and optical characteristics of the examinee's eye are subjectively measured using the target light flux guided to the examinee's eye,
the subjective optometry program, when executed by a processor of the subjective optometry apparatus, causing the subjective optometry apparatus to perform a correction step of correcting inclination of an image plane of an image of the target light flux caused by the target light flux passing through an optical path deviated from an optical axis of the optical element.

### BRIEF DESCRIPTION OF DRAWINGS

Fig. 1 illustrates an exterior view of a subjective optometry apparatus according to an example.
Fig. 2 is a view illustrating a configuration of measurement means.
Fig. 3 is a schematic configuration view when the inside of the subjective optometry apparatus is seen from the front.
Fig. 4 is a schematic configuration view when the inside of the subjective optometry apparatus is seen from the side.
Fig. 5 is a schematic configuration view when the inside of the subjective optometry apparatus is seen from above.
Fig. 6 is a view illustrating a control system of the subjective optometry apparatus.
Fig. 7 is a view illustrating an anterior ocular segment image of an examinee's eye.
Fig. 8 is a view illustrating alignment control.
Fig. 9 is a view for describing inclination of an image plane of an image of a target light flux.
Fig. 10 is a view illustrating the image plane of the image of the target light flux due to the tilting of a display.
Fig. 11 is a view for describing distortion of the target light flux.
Fig. 12 is a view for describing correction of the distortion in target light flux.

### DETAILED DESCRIPTION

### <Outline>

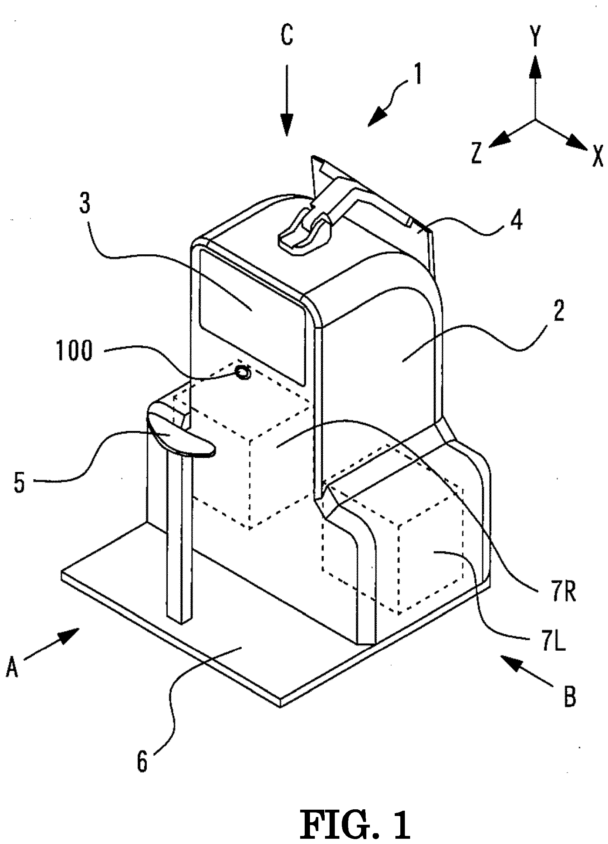

Hereinafter, one of typical embodiments will be described with reference to the drawings. Figs. 1 to 12 are views illustrating a subjective optometry apparatus according to the embodiment. Meanwhile, this disclosure is not limited to the apparatus described in the example. For example, terminal control software (program) for performing functions of the embodiments which will be described below is supplied to a system or an apparatus via a network or various storage media, and a control device (for example, a CPU or the like) of the system or the apparatus can also read the program. Meanwhile, items classified as the following sign "< >" may be used independently of or in relation to each other.

In the following description, a description will be given on the assumption that a depth direction (a front-back direction of an examinee) of the subjective optometry apparatus is a Z direction, a horizontal direction (a left-right direction of the examinee) on a plane which is perpendicular to the depth direction is an X direction, and a vertical direction (an up-down direction of the examinee) on a plane which is perpendicular to the depth direction is a Y direction. In addition, L and R attached to the reference numerals indicate left eye and right eye, respectively.

For example, the subjective optometry apparatus (for example, a subjective optometry apparatus 1) in the present embodiment subjectively measures optical characteristics of an examinee's eye. For example, the subjective optometry apparatus may include a light projecting optical system (for example, a light projecting optical system 30). For example, the light projecting optical system projects a target light flux to the examinee's eye to project a visual target onto the examinee's eye. In addition, for example, the subjective optometry apparatus may include a calibration optical system (for example, a calibration optical system 60 and a subjective measurement optical system 25). For example, the calibration optical system is disposed in an optical path from the light projecting optical system to the examinee's eye and changes the optical characteristics of the target light flux. Also, for example, the subjective optometry apparatus may include an optical element (for example, a concave surface mirror 85) that guides the target light flux of which the optical characteristics have been changed by the calibration optical system to the examinee's eye.

For example, the target light flux passes through the optical path deviated from the optical axis of the optical element and is guided to the examinee's eye. For example, the subjective optometry apparatus in the present embodiment subjectively measures the optical characteristics of the examinee's eye using the image of the target light flux guided to the examinee's eye. In addition, for example the subjective optometry apparatus in the present embodiment subjectively measures the optical characteristics of the examinee's eye using the target light flux guided to the examinee's eye.

Examples of the optical characteristic of the examinee's eye which is subjectively measured may include at least any one of an eye refractive power (for example, at least any one of a spherical power, a cylindrical power, and an astigmatic axis angle), a contrast sensitivity, binocular vision function (for example, at least any one of the amount of oblique position and a stereoscopic function), and the like.

<Light Projecting Optical System>

For example, the light projecting optical system includes a light source (for example, a display 31) that emits the target light flux. In addition, for example, the light projecting optical system may include at least one or more optical elements that guide the target light flux projected from the light source emitting the target light flux to the examinee's eye. For example, a configuration may also be adopted in which a display is used as the light source that projects the target light flux. For example, a liquid crystal display (LCD), an organic electroluminescence (EL), or the like is used as the display. For example, an examination visual target such as a Landolt ring visual target is displayed on the display. In addition, for example, a light source and a digital micromirror device (DMD) may be used as the light source that projects the target light flux. In general, the DMD has high reflectivity and luminance. Therefore, it is possible to maintain the amount of light of the target light flux as compared to a case where a liquid crystal display using polarization is used.

For example, the light source projecting the target light flux may be configured to include a visual target presentation visible light source and a visual target plate. In this case, for example, the visual target plate is a rotatable disc plate, and includes a plurality of visual targets. For example, the plurality of visual targets include a visual target for examination of visual acuity which is used during subjective measurement, and the like. For example, as the visual target for examination of visual acuity, a visual target (visual acuity value 0.1, 0.3, ... , 1.5) is provided for each visual acuity value. For example, the visual target plate is rotated by a motor or the like, and the visual targets are disposed in a switching manner in an optical path through which the target light flux is guided to the examinee's eye. Naturally, a light source other than the light source having the above-described configuration may be used as the light source projecting the target light flux.

For example, the light projecting optical system may include a right eye light projecting optical system and a left eye light projecting optical system which are provided as a pair on the left and right sides respectively. For example, the left eye projecting optical system and the right eye projecting optical system may be configured such that elements configuring the left eye projecting optical system and members configuring the right eye projecting optical system are the same members. In addition, for example, the left eye light projecting optical system and the right eye light projecting optical system may be configured such that at least some of the members configuring the left eye light projecting optical system and the members configuring the right eye light projecting optical system are members different from each other. In addition, for example, the left eye light projecting optical system and the right eye light projecting optical system may be configured such that at least some of the members configuring the left eye light projecting optical system and the members configuring the right eye light projecting optical system are used in common. In addition, for example, the left eye light projecting optical system and the right eye light projecting optical system may be configured such that the members configuring the left eye light projecting optical system and the members configuring the right eye light projecting optical system are separately provided.

### <Calibration Optical System>

For example, the calibration optical system may be configured to change optical characteristics (for example, at least any one of a spherical power, a cylindrical power, a cylindrical axis, polarization characteristics, and the amount of aberration) of the target light flux. For example, as a configuration in which the optical characteristics of the target light flux is changed, a configuration in which an optical element is controlled may be adopted. For example, as the optical element, a configuration may also be adopted in which at least any one of a spherical lens, a cylindrical lens, a cross cylinder lens, a rotary prism, a wavefront modulation element, and the like is used. Naturally, for example, as the optical element, an optical element different from the optical element having the above-described configuration may be used.

For example, the calibration optical system may be configured such that a spherical power of the examinee's eye is calibrated by a presentation position (presenting distance) of the visual target with respect to the examinee's eye is optically changed. In this case, for example, as a configuration in which the presentation position (presenting distance) of the visual target is optically changed, a configuration may also be adopted in which a light source (for example, a display) is moved in an optical axis direction. Further, for example, as a configuration in which the presentation position (presenting distance) of the visual target is optically changed, a configuration may also be adopted in which the optical element (for example, the spherical lens) disposed in the optical path is moved in the optical axis direction. Naturally, the calibration optical system may have a configuration constituted by a configuration in which the optical element is controlled and a configuration in which the optical element disposed in the optical path is moved in the optical axis direction.

For example, the calibration optical system may be configured to change the optical characteristics of the target light flux by disposing the optical element between the optical element for guiding the target light flux to the examinee's eye from the light projecting optical system and the light source of the light projecting optical system and by controlling the optical element. In other words, the calibration optical system may have a configuration of a phantom lens refractometer (phantom calibration optical system) as calibration means. In this case, for example, the target light flux calibrated by the calibration optical system is guided to the examinee's eye through the optical element.

For example, the calibration optical system includes a left eye calibration optical system and a right eye calibration optical system which are provided as a pair on the left and right sides respectively. For example, the left eye calibration optical system and the right eye calibration optical system may be configured such that members configuring the left eye calibration optical system and members configuring the right eye calibration optical system are the same members. In addition, for example, the left eye calibration optical system and the right eye calibration optical system may be configured such that at least some of the members configuring the left eye calibration optical system and the members configuring the right eye calibration optical system are members different from each other. In addition, for example, the left eye calibration optical system and the right eye calibration optical system may be configured such that at least some of the members configuring the left eye calibration optical system and the members configuring the right eye calibration optical system are used in common. In addition, for example, the left eye calibration optical system and the right eye calibration optical system may be configured such that the members configuring the left eye calibration optical system and the members configuring the right eye calibration optical system are separately provided.

### <Optical Element>

For example, the optical element which guides the target light flux calibrated by the calibration optical system to the examinee's eye may be an optical element which guides the target light flux or an image of the target light flux to the examinee's eye so as to have an optically predetermined examination distance. For example, the concave surface mirror may be used for the optical element. For example, by using the concave surface mirror, it becomes possible to optically present the visual target to a predetermined examination distance in subjective examination means, and when presenting the visual target to the predetermined examination distance, it is unnecessary to dispose a member or the like to have an actual distance. According to this, since an extra member and space are not required, it is possible to reduce the size of the apparatus. Naturally, for example, the optical element is not limited to the concave surface mirror. For example, the optical element may have any configuration as long as the optical element guides the target light flux or the image of the target light flux optically to the examinee's eye so as to have the predetermined examination distance. In this case, for example, a lens or the like may be used as the optical element.

### <Correction of Image Plane of Image in Target light Flux>

For example, the subjective optometry apparatus in the present embodiment may include correction means (for example, a control section 70). For example, the correction means may be configured to correct the inclination of the image plane of the image of the target light flux caused by the target light flux passing through the optical path deviated from the optical axis of the optical element. In other words, the correction means may be configured to correct the inclination of the image plane of the image of the target light flux projected onto the examinee's eye fundus.

According to this, it is possible to subjectively measure the optical characteristics of the examinee's eye in a state where the inclination of the image plane of the image of the target light flux is reduced. Therefore, an examiner can perform subjective measurement with respect to examinee's eye with high accuracy.

For example, the correction means may correct at least a part of the image plane of the image at the target light flux. In other words, the correction means may correct a light condensing position of at least a part of the target light flux. In addition, for example, the correction means may correct the entire image plane of the image at the target light flux. In other words, the correction means may correct the entire light condensing position of the target light flux.

For example, the correction means may be configured to correct the inclination of the image plane of the image of the target light flux by changing the angle of the surface of the display with respect to the optical axis. In this case, for example, the light projecting optical system may be configured to have the display, and to emit the target light flux as the visual target is displayed on the display. For example, by the configuration for changing the angle of the surface of the display, the examiner can easily correct the inclination of the image plane of the image of target light flux.

For example, the display may be configured to be capable of being rotationally driven around a rotation axis in which the display extends in the vertical direction (Y direction). For example, as the display is rotated around the rotation axis that extends in the vertical direction (up-down direction), the display is tilted in the horizontal direction (X direction) with respect to the optical axis direction of the light projecting optical system. In other words, for example, the display may be configured to be capable of changing the rotation angle in the horizontal direction (left-right direction) with respect to the optical axis of the light projecting optical system.

For example, the display may be configured to be capable of being rotationally driven around the rotation axis in which the display extends in the horizontal direction (X direction). For example, as the display is rotated around the rotation axis that extends in the horizontal direction (left-right direction), the display is tilted in the vertical direction (Y direction) with respect to the optical axis direction of the light projecting optical system. In other words, for example, the display may be configured to be capable of changing the rotation angle in the vertical direction (up-down direction) with respect to the optical axis of the light projecting optical system.

For example, the display may be configured to be rotatable two-dimensionally. In this case, for example, the display may be configured to be respectively rotationally driven around the rotation axis that extends in the left-right direction and the rotation axis that extends in the up-down direction by driving driving means. In other words, for example, the display may be configured to be capable of changing the rotation angle in the XY direction with respect to the optical axis of the light projecting optical system.

In addition, for example, the correction means may be configured to correct the inclination of the image plane of the image of the target light flux by controlling the driving means and by moving the optical element. In this case, for example, the light projecting optical system may be configured to include a moving optical element which is movable with respect to the optical path of the light projecting optical system, and the driving means for moving the moving optical element with respect to the optical path of the light projecting optical system. Examples of the moving optical element may include a lens, a prism, a mirror, and the like. In addition, for example, any optical element of the light projecting optical system may be used as the moving optical element. In addition, for example, a different member which is provided separately from the optical element of the light projecting optical system may be used as the moving optical element. For example, with a configuration of controlling the driving means and by moving the optical element, the examiner can dispose the optical element at an appropriate position with respect to the examinee's eye and can correct the inclination of the image plane of the image of the target light flux with high accuracy.

### <Correction of Image Plane based on Calibration Power>

For example, the subjective optometry apparatus in the present embodiment may include acquisition means (for example, a control section 70) for acquiring a calibration power of the calibration optical system. In this case, for example, the subjective optometry apparatus may be configured to correct the inclination of the image plane of the image of the target light flux based on the calibration power of the calibration optical system. Accordingly, it is possible to suppress the inclination of the image plane of the image of the target light flux caused by the change in calibration power of the calibration optical system in accordance with the eye refractive power of the examinee's eye. Therefore, an examiner can perform subjective measurement with respect to examinee's eye with high accuracy.

For example, the subjective optometry apparatus may include correction amount setting means (for example, the control section 70) for setting a correction amount for correcting the inclination of the image plane of the image of the target light flux based on the calibration power of the calibration optical system. In addition, for example, the subjective optometry apparatus may include the correction means (for example, the control section 70) for correcting the inclination of the image plane of the image of the target light flux based on the correction amount set by the correction amount setting means. For example, the correction amount setting means may have a configuration in which the correction amount based on the calibration power of the calibration optical system is set in advance. In this case, for example, a correction table based on the calibration power of the calibration optical system may be stored in storage means (for example, a memory 75) and the correction amount may be set by calling the correction amount from the storage means. In addition, for example, the correction amount setting means may have a configuration in which arithmetic operation processing is performed based on the calibration power of the calibration optical system and the correction amount is calculated.

For example, the acquisition means may be configured to acquire the calibration power of the calibration optical system by measuring the eye refractive power of the examinee's eye by an objective measurement optical system (for example, an objective measurement optical system 10) included in the subjective optometry apparatus. In addition, for example, the acquisition means may be configured to acquire the calibration power of the calibration optical system by measuring the eye refractive power of the examinee's eye by a subjective measurement optical system (for example, the objective measurement optical system 25) included in the subjective optometry apparatus. In this case, for example, the eye refractive power may be the eye refractive power acquired at the timing during subjective measurement. In addition, for example, the eye refractive power may be the eye refractive power acquired at the timing during subjective measurement.

For example, the acquisition means may acquire the calibration power of the calibration optical system by receiving the measured eye refractive power of the examinee's eye by the objective measurement optical system or the subjective measurement optical system in an apparatus different from the subjective optometry apparatus. In addition, for example, the acquisition means may acquire the calibration power of the calibration optical system by receiving the eye refractive power of the examinee's eye input by the examiner by operating operation means.

### <Correction of Image Plane based on Positional Shift of Target light Flux with respect to Examinee's eye>

For example, the subjective optometry apparatus in the present embodiment may include a measurement unit (for example, measurement means 7) which accommodates the light projecting optical system. In addition, for example, the subjective optometry apparatus may include shift detection means (for example, the control section 70) for detecting the positional shift of the target light flux with respect to the examinee's eye. In addition, for example, the subjective optometry apparatus may be configured to correct the inclination of the image plane of the image of the target light flux based on the detected positional shift. Accordingly, it is possible to suppress the inclination of the image plane of the image of the target light flux caused by the positional shift of the target light flux with respect to the examinee's eye. Therefore, an examiner can perform subjective measurement with respect to examinee's eye with high accuracy.

For example, the correction based on the positional shift may be correction in which the positional shift is directly used, or may be correction in which position information of the measurement unit moved based on the positional shift (correction in which the positional shift is indirectly used).

For example, the shift detection means which uses an alignment index light projecting optical system (for example, a first index projection optical system 45 and a second index projection optical system 46) which projects alignment light to the examinee's eye and forms an alignment index on the periphery of the cornea, can be mentioned. In this case, for example, the shift detection means may be configured to detect a relative position of the examinee's eye and a projection position (optical axis of the light projecting optical system) of the target light flux by detecting the alignment state based on the alignment index captured by an anterior ocular segment observation optical system (for example, the observation optical system 50). Further, for example, the shift detection means may be configured to detect the position of the pupil from the anterior ocular segment front image captured by the anterior ocular segment observation optical system and detect the relative position of the detected position of the pupil and the projection position (optical axis of the light projecting optical system) of the target light flux.

For example, the subjective optometry apparatus may include the correction amount setting means (for example, the control section 70) for setting the correction amount for correcting the inclination of the image plane of the image of the target light flux based on the positional shift. In addition, for example, the subjective optometry apparatus may include the correction means (for example, the control section 70) for correcting the inclination of the image plane of the image of the target light flux based on the correction amount set by the correction amount setting means. For example, the correction amount setting means may have a configuration in which the correction amount based on the positional shift amount is set in advance. In this case, for example, the correction table based on the positional shift may be stored in the storage means and the correction amount may be set by calling the correction amount from the storage means. In addition, for example, the correction amount setting means may have a configuration in which the arithmetic operation processing is performed based on the positional shift amount and the correction amount is calculated.

For example, in a case of performing the correction using the position information of the measurement unit moved based on the positional shift, the subjective optometry apparatus may include moving means for moving the measurement means unit based on the detection result detected by the shift detection means. In addition, for example, the subjective optometry apparatus may include position information acquisition means (for example, the control section 70) for acquiring the position information of the measurement unit. In addition, for example, the subjective optometry apparatus may be configured to correct the inclination of the image plane of the image of the target light flux based on the position information. According to this, it is possible to suppress the inclination of the image plane of the image of the target light flux caused when performing the alignment of the examinee's eye and the measurement means. Therefore, an examiner can perform subjective measurement with respect to examinee's eye with high accuracy.

For example, the position information acquisition means may have a configuration in which the position information of the measurement unit can be acquired. For example, as a configuration for acquiring the position information of the measurement unit, a configuration for detecting the movement (for example, position information of the measurement unit) of the measurement unit may be adopted. In addition, as a configuration for detecting the position information of the measurement unit, a configuration for detecting the position of the measurement unit or a configuration for detecting the movement amount of the measurement unit may be adopted. In addition, the position information of the measurement unit may be the position information of the entire measurement unit or the position information of at least one member of the light projecting optical system accommodated in the measurement unit. In addition, the position information of the measurement unit may be position information of an optical element (for example, a deflection mirror 81) moved together with the measurement unit in the subjective optometry apparatus 1. In this case, the optical element which is moved together with the measurement unit may be configured to move integrally with the measurement unit.

In addition, for example, as a configuration for acquiring the position information of the measurement unit, a configuration may be adopted in which relative position information between the examinee's eye (for example, the cornea apex position or the position of the pupil of the examinee's eye) and the measurement unit is acquired. In addition, for example, in a case of acquiring the relative position information between the examinee's eye and the measurement unit, the position information acquisition means may be configured to acquire the relative position information by detecting the position of the examinee's eye and the position of the measurement unit, respectively. In addition, for example, in a case of acquiring the relative position information between the examinee's eye and the measurement unit, the position information acquisition means may be configured to acquire the relative position information by detecting the position of the measurement unit. In this case, for example, the position of the measurement unit may be stored in the storage means in advance. In addition, for example, the position information acquisition means may be configured to acquire the relative position information by detecting the movement amount of the measurement unit. In this case, for example, the movement amount of the measurement unit moved from a preset initial position may be detected. In addition, the position information of the measurement unit may be the position information of the entire measurement unit or the position information of at least one member of the light projecting optical system accommodated in the measurement unit.

In addition, for example, as a configuration for acquiring the position information of the measurement unit, a configuration for acquiring the position information of the measurement unit by acquiring the relative position information of the optical element and the measurement unit. In addition, for example, in a case of acquiring the relative position information between the optical element and the measurement unit, the position information acquisition means may be configured to acquire the relative position information by detecting the position of the optical element and the position of the measurement unit, respectively. In addition, for example, in a case of acquiring the relative position information between the optical element and the measurement unit, the position information acquisition means may be configured to acquire the relative position information by detecting the position of the measurement unit. In this case, for example, the position of the optical element may be stored in the storage means in advance. In addition, for example, the position information acquisition means may be configured to acquire the relative position information by detecting the movement amount of the measurement unit. In this case, for example, the movement amount of the measurement unit moved from a preset initial position may be detected. In addition, the position information of the measurement unit may be the position information of the entire measurement unit or the position information of at least one member of the light projecting optical system accommodated in the measurement unit.

### < Correction of Distortion in Target light Flux>

For example, the correction means may be configured to correct distortion of the target light flux caused by the target light flux passing through the optical path deviated from the optical axis of the optical element. In other words, the correction means may be configured to correct the distortion of the target light flux projected onto the examinee's eye fundus. According to this, it is possible to subjectively measure the optical characteristics of the examinee's eye in a state where the distortion of the target light flux is reduced. Therefore, an examiner can perform subjective measurement with respect to examinee's eye with high accuracy.

For example, the correction means may correct at least a part of the distortion of the target light flux, or correct the whole distortion of the target light flux. For example, the correction means may be configured to correct the distortion of the target light flux by deforming the visual target displayed on the display (for example, the display 31). In this case, for example, the light projecting optical system may be configured to have the display, and to emit the target light flux as the visual target is displayed on the display.

In a case of performing the correction using the display, for example, the correction means may change the size in the longitudinal direction of the visual target displayed on the display. In addition, for example, the correction means may change the size in the longitudinal direction of the visual target displayed on the display. In addition, for example, the correction means may move the visual target in the visual target displayed on the display. In other words, the correction means may be configured to perform at least any one processing among the change in size in the longitudinal direction, the change in size in the lateral direction, and the movement of the visual target, in the visual target displayed on the display. For example, by the configuration for deforming the visual target displayed on the display, the examiner can easily correct the distortion of the target light flux.

In addition, for example, the correction means may be configured to correct the distortion of the target light flux by controlling the driving means and by moving the optical element. In this case, for example, the light projecting optical system may be configured to include the moving optical element which is movable with respect to the optical path of the light projecting optical system, and the driving means for moving the moving optical element with respect to the optical path of the light projecting optical system. Examples of the moving optical element may include a lens, a prism, a mirror, and the like. In addition, for example, as the moving optical element, any optical element of the light projecting optical system may be used, or a different member which is provided separately from the optical element of the light projecting optical system may be used. For example, by controlling the driving means and by moving the optical element, the examiner can correct the distortion of the target light flux with high accuracy.

### <Correction of Distortion based on Calibration Power>

For example, the subjective optometry apparatus in the present embodiment may include acquisition means (for example, a control section 70) for acquiring a calibration power of the calibration optical system. In this case, for example, the subjective optometry apparatus may be configured to correct the distortion of the target light flux based on the calibration power of the calibration optical system. According to this, it is possible to suppress the distortion of the image of the target light flux caused by the change in calibration power of the calibration optical system in accordance with the eye refractive power of the examinee's eye. Therefore, an examiner can perform subjective measurement with respect to examinee's eye with high accuracy.

For example, the subjective optometry apparatus may include the correction amount setting means (for example, the control section 70) for setting the correction amount for correcting the distortion of the target light flux based on the calibration power of the calibration optical system. In addition, for example, the subjective optometry apparatus may include the correction means (for example, the control section 70) for correcting the distortion of the image of the target light flux based on the correction amount set by the correction amount setting means. For example, the correction amount setting means may have a configuration in which the correction amount based on the calibration power of the calibration optical system is set in advance. In this case, for example, a correction table based on the calibration power of the calibration optical system may be stored in the storage means (for example, a memory 75) and the correction amount may be set by calling the correction amount from the storage means. In addition, for example, the correction amount setting means may have a configuration in which arithmetic operation processing is performed based on the calibration power of the calibration optical system and the correction amount is calculated.

For example, the acquisition means may acquire the calibration power of the calibration optical system by measuring the eye refractive power of the examinee's eye by an objective measurement optical system (for example, an objective measurement optical system 10) included in the subjective optometry apparatus. In addition, for example, the acquisition means may acquire the calibration power of the calibration optical system by measuring the eye refractive power of the examinee's eye by a subjective measurement optical system (for example, the objective measurement optical system 25) included in the subjective optometry apparatus. In this case, for example, the eye refractive power may be the eye refractive power acquired at the timing during subjective measurement. In addition, for example, the eye refractive power may be the eye refractive power acquired at the timing during subjective measurement.

For example, the acquisition means may acquire the calibration power of the calibration optical system by receiving the measured eye refractive power of the examinee's eye by the objective measurement optical system or the subjective measurement optical system in an apparatus different from the subjective optometry apparatus. In addition, for example, the acquisition means may acquire the calibration power of the calibration optical system by receiving the eye refractive power of the examinee's eye input by the examiner by operating operation means.

### <Correction of Distortion based on Positional Shift of Target light Flux with respect to Examinee's eye>

For example, the subjective optometry apparatus in the present embodiment may include a measurement unit (for example, the measurement means 7) which accommodates the light projecting optical system. In addition, for example, the subjective optometry apparatus may include shift detection means (for example, the control section 70) for detecting the positional shift of the target light flux with respect to the examinee's eye. In addition, for example, the subjective optometry apparatus may be configured to correct the distortion of the image of the target light flux based on the detected positional shift. Accordingly, it is possible to suppress the distortion of the image of the target light flux caused by the positional shift of the target light flux with respect to the examinee's eye. Therefore, an examiner can perform subjective measurement with respect to examinee's eye with high accuracy.

For example, the correction based on the positional shift may be correction in which the positional shift is directly used, or may be correction in which position information of the measurement unit moved based on the positional shift (correction in which the positional shift is indirectly used).

For example, the shift detection means which uses an alignment index light projecting optical system (for example, a first index projection optical system 45 and a second index projection optical system 46) which projects alignment light to the examinee's eye and forms an alignment index on the periphery of the cornea, can be mentioned. In this case, for example, the shift detection means may be configured to detect a relative position of the examinee's eye and a projection position (optical axis of the light projecting optical system) of the target light flux by detecting the alignment state based on the alignment index captured by an anterior ocular segment observation optical system (for example, the observation optical system 50). Further, for example, the shift detection means may be configured to detect the position of the pupil from the anterior ocular segment front image captured by the anterior ocular segment observation optical system and detect the relative position of the detected position of the pupil and the projection position (optical axis of the light projecting optical system) of the target light flux.

For example, the subjective optometry apparatus may include the correction amount setting means (for example, the control section 70) for setting the correction amount for correcting the distortion of the image of the target light flux based on the positional shift. In addition, for example, the subjective optometry apparatus may include the correction means (for example, the control section 70) for correcting the distortion of the image of the target light flux based on the correction amount set by the correction amount setting means. For example, the correction amount setting means may have a configuration in which the correction amount based on the positional shift amount is set in advance. In this case, for example, the correction table based on the positional shift amount may be stored in the storage means and the correction amount may be set by calling the correction amount from the storage means. In addition, for example, the correction amount setting means may have a configuration in which the arithmetic operation processing is performed based on the positional shift amount and the correction amount is calculated.

For example, in a case of performing the correction using the position information of the measurement unit moved based on the positional shift, the subjective optometry apparatus may include moving means for moving the measurement means unit based on the detection result detected by the shift detection means. In addition, for example, the subjective optometry apparatus may include position information acquisition means (for example, the control section 70) for acquiring the position information of the measurement unit. In addition, for example, the subjective optometry apparatus may be configured to correct the distortion of the image of the target light flux based on the position information. According to this, it is possible to suppress the distortion of the target light flux caused when performing the alignment of the examinee's eye and the measurement means. Therefore, an examiner can perform subjective measurement with respect to examinee's eye with high accuracy.

For example, the position information acquisition means may have a configuration in which the position information of the measurement unit can be acquired. For example, as a configuration for acquiring the position information of the measurement unit, a configuration for detecting the movement (for example, position information of the measurement unit) of the measurement unit may be adopted. In addition, as a configuration for detecting the position information of the measurement unit, a configuration for detecting the position of the measurement unit or a configuration for detecting the movement amount of the measurement unit may be adopted. In addition, the position information of the measurement unit may be the position information of the entire measurement unit or the position information of at least one member of the light projecting optical system accommodated in the measurement unit. In addition, the position information of the measurement unit may be position information of an optical element (for example, a deflection mirror 81) moved together with the measurement unit in the subjective optometry apparatus 1. In this case, the optical element which is moved together with the measurement unit may be configured to move integrally with the measurement unit.

In addition, for example, as a configuration for acquiring the position information of the measurement unit, a configuration may be adopted in which relative position information between the examinee's eye (for example, the cornea apex position or the position of the pupil of the examinee's eye) and the measurement unit is acquired. In addition, for example, in a case of acquiring the relative position information between the examinee's eye and the measurement unit, the position information acquisition means may be configured to acquire the relative position information by detecting the position of the examinee's eye and the position of the measurement unit, respectively. In addition, for example, in a case of acquiring the relative position information between the examinee's eye and the measurement unit, the position information acquisition means may be configured to acquire the relative position information by detecting the position of the measurement unit. In this case, for example, the position of the measurement unit may be stored in the storage means in advance. In addition, for example, the position information acquisition means may be configured to acquire the relative position information by detecting the movement amount of the measurement unit. In this case, for example, the movement amount of the measurement unit moved from a preset initial position may be detected. In addition, the position information of the measurement unit may be the position information of the entire measurement unit or the position information of at least one member of the light projecting optical system accommodated in the measurement unit.

In addition, for example, as a configuration for acquiring the position information of the measurement unit, a configuration for acquiring the position information of the measurement unit by acquiring the relative position information of the optical element and the measurement unit. In addition, for example, in a case of acquiring the relative position information between the optical element and the measurement unit, the position information acquisition means may be configured to acquire the relative position information by detecting the position of the optical element and the position of the measurement unit, respectively. In addition, for example, in a case of acquiring the relative position information between the optical element and the measurement unit, the position information acquisition means may be configured to acquire the relative position information by detecting the position of the measurement unit. In this case, for example, the position of the optical element may be stored in the storage means in advance. In addition, for example, the position information acquisition means may be configured to acquire the relative position information by detecting the movement amount of the measurement unit. In this case, for example, the movement amount of the measurement unit moved from a preset initial position may be detected. In addition, the position information of the measurement unit may be the position information of the entire measurement unit or the position information of at least one member of the light projecting optical system accommodated in the measurement unit.

### <Example>

Hereinafter, the subjective optometry apparatus of the present example will be described. For example, the subjective optometry apparatus may include subjective measurement means. In addition, for example, the subjective optometry apparatus may include objective measurement means. In addition, in the present example, the subjective optometry apparatus provided with both the subjective measurement means and the objective measurement means will be described as an example.

Fig. 1 illustrates an exterior view of the subjective optometry apparatus 1 according to the present example. For example, the subjective optometry apparatus 1 includes a housing 2, a presentation window 3, a monitor 4, a chin mount 5, a base 6, an anterior ocular segment image capture optical system 100, and the like. For example, the housing 2 includes the measurement means 7 on the inside thereof (details thereof will be described later). For example, the presentation window 3 is used to present the visual target to examinee. For example, the target light flux from the measurement means 7 is projected onto a examinee's eye E of the examinee via the presentation window 3.

For example, the monitor (display) 4 displays the optical characteristics result (for example, spherical refractivity S, cylindrical refractivity C, astigmatic axis angle A, prism value Δ and the like) of the examinee's eye E. For example, the monitor 4 is a touch panel. In other words, in the present example, the monitor 4 functions as an operation section (controller). For example, the signal that corresponds to an operation instruction input from the monitor 4 is output to the control section 70 which will be described later. In addition, the monitor 4 may not be a touch panel type, or may be configured to separately provide the monitor 4 and the operation section. For example, in this case, the operation section may be configured to use at least one operation means, such as a mouse, a joystick, or a keyboard.

For example, the monitor 4 may be a display mounted on the housing 2, or may be a display connected to the housing 2. For example, in this case, a configuration using the display of a personal computer may be used. In addition, for example, a plurality of displays may be used together.

For example, the distance between the examinee's eye E and the subjective optometry apparatus 1 is kept constant by the chin mount 5. In the present example, the configuration using the chin mount 5 is used as an example to keep the distance between the examinee's eye E and the subjective optometry apparatus 1 constant, but the invention is not limited thereto. For example, in the present example, a configuration may be adopted in which a forehead protector, a face protector, and the like are used in order to keep the distance between the examinee's eye E and the subjective optometry apparatus 1 constant. For example, the chin mount 5 and the housing 2 are fixed to the base 6.

For example, the anterior ocular segment image capture optical system 100 is configured with an image capture element and a lens which are not illustrated in the drawing. For example, the anterior ocular segment image capture optical system 100 is used to capture an image of the face of the examinee.

### <Measurement Means>

For example, the measurement means 7 includes a left eye measurement means 7L and a right eye measurement means 7R. In other words, the subjective optometry apparatus 1 in the present example includes a pair of left and right subjective measurement means and a pair of left and right objective measurement means. In addition, in the present example, the subjective optometry apparatus 1 includes either one of the left eye measurement means 7L and the right eye measurement means 7R, and by moving the means in the left-right direction, the target light flux may be projected onto each of a left examinee's eye EL and a right examinee's eye ER. For example, the left eye measurement means 7L and the right eye measurement means 7R in the present example include the same member. Naturally, the left eye measurement means 7L and the right eye measurement means 7R may be configured such that at least some members thereof are different from each other.

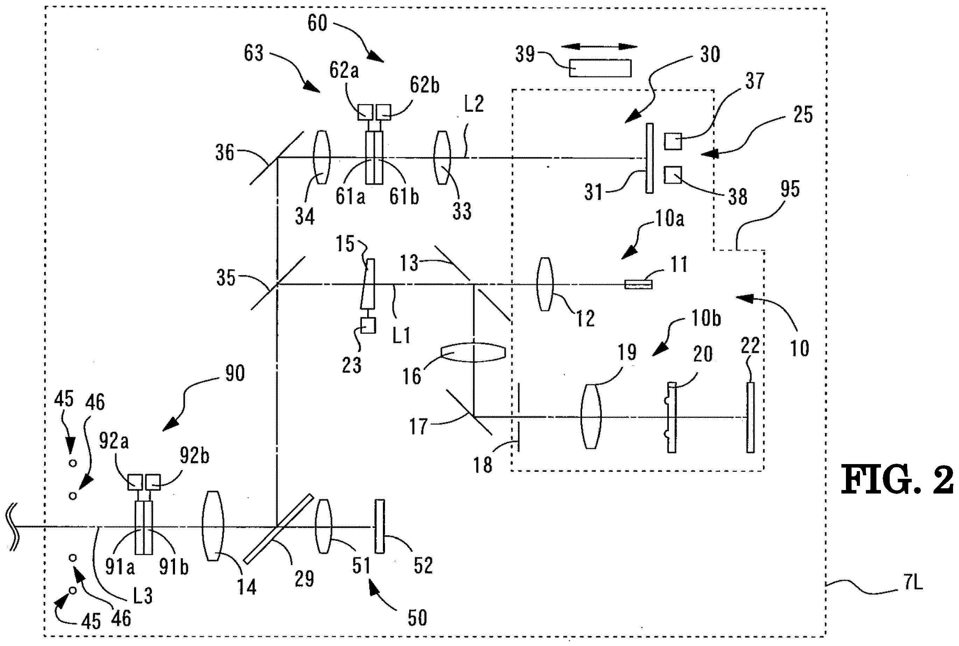

Fig. 2 is a view illustrating a configuration of the measurement means 7. For example, in the present example, the left eye measurement means 7L is described as an example. In addition, since the right eye measurement means 7R has the same configuration as that of the left eye measurement means 7L, the description thereof will be omitted. For example, the left eye measurement means 7L includes the subjective measurement optical system 25, the objective measurement optical system 10, the first index projection optical system 45, the second index projection optical system 46, and the observation optical system 50.

### <Subjective Optical System>

For example, the subjective measurement optical system 25 is used as a part of the configuration of the subjective measurement means for subjectively measuring optical characteristics of the examinee's eye E (details thereof will be described later). Examples of the optical characteristics of the examinee's eye E include an eye refractive power, a contrast sensitivity, a binocular vision function (for example, the amount of oblique position, a stereoscopic function, and the like), and the like. In addition, in the present example, an example of the subjective measurement means for measuring the eye refractive power of the examinee's eye E will be described. For example, the subjective measurement optical system 25 includes the light projecting optical system (visual target projection system) 30, the calibration optical system 60, and a correction optical system 90.

For example, the light projecting optical system 30 projects the target light flux to the examinee's eye E. For example, the light projecting optical system 30 includes the display 31, a projection lens 33, a projection lens 34, a reflecting mirror 36, a dichroic mirror 35, a dichroic mirror 29, and an objective lens 14. For example, the target light flux projected from the display 31 is projected onto the examinee's eye E through the optical element in order of the projection lens 33, the projection lens 34, the reflecting mirror 36, the dichroic mirror 35, the dichroic mirror 29, and the objective lens 14.

For example, the display 31 is provided with a driving mechanism 37 (for example, a motor) for rotating (tilting) the display 31 in the up-down direction. For example, the display 31 rotates around an axis that extends in the left-right direction from the center of the display 31 by the driving mechanism 37. In addition, the display 31 may rotate around an axis that extends in the left-right direction from a position different from the center of the display 31. According to this, the display 31 is tilted upward and downward with respect to an optical axis L2 direction. Further, for example, the display 31 is provided with a driving mechanism 38 (for example, a motor) for rotating (panning) the display 31 in the left-right direction. For example, the display 31 rotates around an axis that extends in the up-down direction from the center of the display 31 by the driving mechanism 38. In addition, the display 31 may rotate around the axis that extends in the up-down direction from a position different from the center of the display 31. According to this, the display 31 is tilted leftward and rightward with respect to the optical axis L2 direction.

For example, an examination visual target, such as a Landolt ring visual target, a fixation target for fixedly viewing the examinee's eye E, and the like are displayed on the display 31. For example, the target light flux from the display 31 is projected to the examinee's eye E. For example, in the present example, the following description will be given by take a case where a liquid crystal display (LCD) is used as the display 31 as an example. In addition, as a display, an organic electro luminescence (EL) display, a plasma display, or the like can also be used.

For example, the calibration optical system 60 is disposed in the optical path of the light projecting optical system 30. For example, the calibration optical system 60 changes the optical characteristics of the target light flux. For example, the calibration optical system 60 includes an astigmatism calibration optical system 63 and a driving mechanism 39. For example, the astigmatism calibration optical system 63 is disposed between the projection lens 33 and the projection lens 34. For example, the astigmatism calibration optical system 63 is used for calibrating a cylindrical power, a cylindrical axis (astigmatic axis), and the like of the examinee's eye E. For example, the astigmatism calibration optical system 63 is configured with two positive cylindrical lenses 61a and 61b having the same focal length. The cylindrical lenses 61a and 61b are independently rotated around an optical axis L2 by the driving of respective rotation mechanisms 62a and 62b. Meanwhile, in the present example, the astigmatism calibration optical system 63 has been described using an example of a configuration in which the two positive cylindrical lenses 61a and 61b are used, but the invention is not limited thereto. The astigmatism calibration optical system 63 may be configured to be capable of calibrating a cylindrical power, a cylindrical axis, and the like. In this case, a configuration may also be adopted in which the calibration lens is inserted into and removed from the optical path of the light projecting optical system 30.

For example, the driving mechanism 39 is configured with a motor and a slide mechanism. For example, by the driving mechanism 39, the display 31 is integrally moved in the direction of the optical axis L2. For example, the presentation position (presenting distance) of the visual target with respect to the examinee's eye E is optically changed by the movement of the display 31 during the subjective measurement, and a spherical refractive power of the examinee's eye E is calibrated. In other words, the calibration optical system of the spherical power is configured by the movement of the display 31. In addition, for example, fogging is applied to the examinee's eye E by the movement of the display 31 during the objective measurement. Meanwhile, the calibration optical system of the spherical power is not limited thereto. For example, the calibration optical system of the spherical power includes a large number of optical elements, and may be configured to perform calibration by the optical elements being disposed in the optical path. In addition, for example, the calibration optical system of the spherical power may be configured to move the lens disposed in the optical path in the optical axis direction.

Meanwhile, in the present example, an example of the calibration optical system for calibrating a spherical power, a cylindrical power, and a cylindrical axis has been described, but the invention is not limited thereto. For example, the calibration optical system for calibrating a prism value may be provided. The calibration optical system for the prism value is provided, and thus, it is possible to perform calibration such that the target light flux is projected onto the examinee's eye E even when the examinee has heterophoria.

Meanwhile, in the present example, a description has been given of an example of a configuration in which the astigmatism calibration optical system 63 for calibrating the cylindrical power and the cylindrical axis (astigmatic axis) and the calibration optical system (for example, the driving means 39) for calibrating the spherical power are separately provided, but the invention is not limited thereto. For example, as the calibration optical system, a configuration may be adopted in which a calibration optical system for calibrating a spherical power, a cylindrical power, and an astigmatic axis is provided. In other words, the calibration optical system in the present example may be an optical system for modulating the wavefront. In addition, for example, the calibration optical system may be an optical system that calibrats a spherical power, a cylindrical power, an astigmatic axis, and the like. In this case, for example, the calibration optical system may be configured to include a lens disc on which a large number of optical elements (a spherical lens, a cylindrical lens, a dispersing prism, and the like) are disposed on the same circumference. The lens disc is rotationally controlled by the driving section (an actuator, a stepping motor, and the like), and the optical element (for example, a cylindrical lens, a cross cylinder lens, a rotary prism, and the like) desired by the examiner is disposed on the optical axis L2 at the rotation angle desired by the examiner. For example, the switching of the optical element disposed on the optical axis L2, and the like may be performed by the operation of the monitor 4 or the like.

The lens disc is configured with one lens disc or a plurality of lens discs. In a case where a plurality of lens discs are disposed, the driving section that corresponds to each of the lens discs is provided. For example, as a lens disc group, each of the lens discs has an opening (or a 0D lens) and a plurality of optical elements. As a type of each of the lens discs, a spherical lens disc having a plurality of spherical lenses with different frequencies, a cylindrical lens disc having a plurality of cylindrical lenses with different frequencies, and an auxiliary lens disc having a plurality of types of auxiliary lenses are representative. At least one of a red filter and a green filter, a prism, a cross cylinder lens, a polarizing plate, a Maddox lens, and an autocross cylinder lens is disposed on the auxiliary lens disc. In addition, the cylindrical lens is rotatably disposed around the optical axis L2 by the driving section, and the rotary prism and the cross cylinder lens may be disposed to be rotatable around each of the optical axes by the driving section.

For example, the correction optical system 90 is disposed between the objective lens 14 and the deflection mirror 81 which will be described later. For example, the correction optical system 90 is used for correcting optical aberrations (for example, astigmatism) generated in the subjective measurement. For example, the correction optical system 90 is configured with two positive cylindrical lenses 91a and 91b having the same focal length. For example, the correction optical system 90 corrects the astigmatism by adjusting the cylindrical power and the astigmatic axis. Each of the cylindrical lens 91a and the cylindrical lens 91b is independently rotated around an optical axis L3 by driving the rotation mechanisms 92a and 92b, respectively. In addition, in the present example, the configuration using two positive cylindrical lenses 91a and 91b has been described as an example of the correction optical system 90, but the present invention is not limited thereto. The correction optical system 90 may have any configuration as long as the configuration can calibrate the astigmatism. In this case, for example, the correction lens may be inserted into and removed from the optical axis L3.

In addition, in the present example, the configuration in which the correction optical system 90 is disposed separately from the calibration optical system 60 has been described as an example, but the present invention is not limited thereto. For example, the calibration optical system 60 may be configured to also serve as the correction optical system 90. In this case, the cylindrical power of the examinee's eye E and the cylindrical axis (astigmatic axis) are corrected in accordance with the amount of astigmatism. In other words, the calibration optical system 60 is driven so as to calibrate the cylindrical power considering (correcting) the astigmatism amount or the astigmatic axis. For example, by using both the calibration optical system 60 and the correction optical system 90, complicated control is not required, and thus, it is possible to correct the optical aberration with a simple configuration. In addition, for example, by using both the calibration optical system 60 and the correction optical system 90, it is not necessary to separately provide a correction optical system for the optical aberration, and thus, it is possible to correct the optical aberration with a simple configuration.

### <Objective Optical System>

For example, the objective measurement optical system 10 is used as a part of a configuration of the objective measurement means for objectively measuring the optical characteristics of the examinee's eye (details thereof will be described later). Examples of the optical characteristics of the examinee's eye E include an eye refractive power, an ocular axial length, a cornea shape, and the like. In the example, an example of the objective measurement means for measuring an eye refractive power of the examinee's eye E will be described. For example, the objective measurement optical system 10 includes a projection optical system 10a, a light receiving optical system 10b, and a correction optical system 90.

For example, the projection optical system (light projecting optical system) 10a projects a spot-shaped measurement index onto the fundus of the examinee's eye E through the pupil center portion of the examinee's eye E. For example, the light receiving optical system 10b extracts fundus reflected light reflected from the fundus in a ring shape through a pupil peripheral portion, and causes a two-dimensional image capture element 22 to capture a ring-shaped fundus reflected image.

For example, the projection optical system 10a includes a measurement light source 11, a relay lens 12, a hole mirror 13, a prism 15, a driving section (motor) 23, a dichroic mirror 35, a dichroic mirror 29, and an objective lens 14 which are disposed on an optical axis L1 of the objective measurement optical system 10. For example, the prism 15 is a luminous flux deflection member. For example, the driving section 23 rotationally drives the prism 15 around the optical axis L1. For example, the light source 11 has a conjugate relationship with the fundus of the examinee's eye E. Further, the hole portion of the hole mirror 13 has a conjugate relationship with the pupil of the examinee's eye E. For example, the prism 15 is disposed at a position away from the position conjugated with the pupil of the examinee's eye E, and the luminous flux to pass through the prism is eccentric with the optical axis L1. Meanwhile, a configuration may also be adopted in which a parallel plane plate is obliquely disposed on the optical axis L1 as the luminous flux deflection member instead of the prism 15.

For example, the dichroic mirror 35 is common to the optical path of the subjective measurement optical system 25 and the optical path of the objective measurement optical system 10. In other words, for example, the dichroic mirror 35 has the optical axis L2 of the subjective measurement optical system 25 and the optical axis L1 of the objective measurement optical system 10 as the same axis. For example, the dichroic mirror 29 which is an optical path branching member reflects the luminous flux of the subjective measurement optical system 25 and the measurement light of the projection optical system 10a, and guides the reflected luminous flux and measurement light to the examinee's eye E.

For example, the light receiving optical system 10b uses the objective lens 14, the dichroic mirror 29, the dichroic mirror 35, the prism 15, and the hole mirror 13 in common with the projection optical system 10a, and includes a relay lens 16 disposed in the optical path in the reflection direction of the hole mirror 13, a mirror 17, a light receiving diaphragm 18 disposed in the optical path in the reflection direction of the mirror 17, a collimator lens 19, a ring lens 20, and the two-dimensional image capture element 22, such as a CCD. For example, the light receiving diaphragm 18 and the two-dimensional image capture element 22 has a conjugate relationship with the fundus of the examinee's eye E. For example, the ring lens 20 is configured with a lens portion formed in a ring shape and a light shielding portion obtained by performing coating for light shielding in a region other than the lens portion, and has an optically conjugate positional relationship with the pupil of the examinee's eye E. For example, an output from the two-dimensional image capture element 22 is input to the control section 70.

For example, the dichroic mirror 29 reflects the reflected light of the measurement light from the projection optical system 10a guided to the fundus of the examinee's eye E toward the light receiving optical system 10. In addition, for example, the dichroic mirror 29 transmits anterior ocular segment observation light and alignment light, and guides the transmitted light to the observation optical system 50. For example, the dichroic mirror 35 reflects the reflected light of the measurement light from the projection optical system 10a guided to the fundus of the examinee's eye E toward the light receiving optical system 10.

Meanwhile, the objective measurement optical system 10 is not limited to the above-described objective measurement optical system, and it is possible to use a well-known objective measurement optical system configured to project a ring-shaped measurement index onto the fundus from the pupil peripheral portion, to extract the fundus reflected light from the pupil center portion, and to cause the two-dimensional image capture element 22 to receive light of the ring-shaped fundus reflected image.

Meanwhile, the objective measurement optical system 10 is not limited to the above-described objective measurement optical system, and may be a measurement optical system including a light projecting optical system which projects the measurement light toward the fundus of the examinee's eye E and a light receiving optical system in which the reflected light acquired by the reflection of the measurement light from the fundus is received by a light receiving element. For example, an eye refractive power measurement optical system may be configured to include a Shack Hartman sensor. Naturally, an apparatus using another measurement method may be used (for example, an apparatus of a phase difference system which projects a slit).

For example, the light source 11 of the projection optical system 10a, and the light receiving diaphragm 18, the collimator lens 19, the ring lens 20, and the two-dimensional image capture element 22 of the light receiving optical system 10b can be integrally moved in the optical axis direction. In the present example, for example, the light source 11 of the projection optical system 10a and the light receiving diaphragm 18, the collimator lens 19, the ring lens 20, and the two-dimensional image capture element 22 of the light receiving optical system 10b are integrally moved in the direction of the optical axis L1 by the driving mechanism 39 that drives the display 31. In other words, the display 31, the light source 11 of the projection optical system 10a, the light receiving diaphragm 18, the collimator lens 19, the ring lens 20, and the two-dimensional image capture element 22 of the light receiving optical system 10b are integrally moved as a driving unit 95 in synchronization with each other. Naturally, a configuration in which the components are separately driven may also be adopted.

For example, the driving unit 95 moves a part of the objective measurement optical system 10 in the optical axis direction such that an external ring luminous flux is incident on the two-dimensional image capture element 22 with respect to each longitudinal direction. In other words, a part of the objective measurement optical system 10 is moved in the direction of the optical axis L1 in accordance with a spherical refractive error (spherical refractive power) of the examinee's eye E, such that the spherical refractive error is corrected and the light source 11, the light receiving diaphragm 18, and the two-dimensional image capture element 22 are optically conjugated with the fundus of the examinee's eye E. For example, the position of the driving mechanism 39 to be moved is detected by a potentiometer not illustrated in the drawing. Meanwhile, the hole mirror 13 and the ring lens 20 are disposed so as to be conjugated with the pupil of the examinee's eye E with a fixed magnification, regardless of the amount of movement of the driving unit 95.

In the above-described configuration, measurement luminous flux emitted from the light source 11 forms a spot-shaped point light source image on the fundus of the examinee's eye E through the relay lens 12, the hole mirror 13, the prism 15, the dichroic mirror 35, the dichroic mirror 29, and the objective lens 14. At this time, a pupil projection image (projected luminous flux on the pupil) of the hole portion in the hole mirror 13 is eccentrically rotated at high speed by the prism 15 rotating around the optical axis. The point light source image projected onto the fundus is reflected and scattered, is emitted from the examinee's eye E, is condensed by the objective lens 14, and is condensed again at the position of the light receiving diaphragm 18 through the dichroic mirror 29, the dichroic mirror 35, the prism 15 which rotates at high speed, the hole mirror 13, the relay lens 16, and the mirror 17, thereby forming a ring-shaped image on the two-dimensional image capture element 22 by the collimator lens 19 and the ring lens 20.

For example, the prism 15 is disposed in an optical path which is common to the projection optical system 10a and the light receiving optical system 10b. For example, a reflected luminous flux from the fundus passes through the prism 15 which is the same as that of the projection optical system 10a, and thus, backward scanning is performed as if there is no eccentricity of the projected luminous flux and the reflected luminous flux (received luminous flux) on the pupil in the subsequent optical systems.

For example, the correction optical system 90 also serves as the subjective measurement optical system 25. Naturally, a configuration may also be adopted in which a correction optical system used in the objective measurement optical system 10 is separately provided.

### <First Index Projection Optical System and Second Index Projection Optical System>

For example, in the present example, the first index projection optical system 45 and the second index projection optical system 46 are disposed between the correction optical system 90 and the deflection mirror 81. Naturally, the arrangement position of the first index projection optical system 45 and the second index projection optical system 46 are not limited thereto. For example, the first index projection optical system 45 and the second index projection optical system 46 may be provided in a cover of the housing 2. For example, in this case, the first index projection optical system 45 and the second index projection optical system 46 are arranged around the presentation window 3.

For example, in the first index projection optical system 45, a plurality of infrared light sources are disposed on the concentric circle around the optical axis L3 at intervals of 45 degrees, and are disposed so as to be bilaterally symmetrical to each other with a vertical plane passing through the optical axis L3 therebetween. For example, the first index projection optical system 45 emits near infrared light for projecting an alignment index onto the cornea of the examinee's eye E. For example, the second index projection optical system 46 includes six infrared light sources which are disposed at a position different from the position of the first index projection optical system 45. In this case, the first index projection optical system 45 is configured to project an index at an infinite distance onto the cornea of the examinee's eye E from the left-right direction, and the second index projection optical system 46 is configured to project an index at a finite distance onto the cornea of the examinee's eye E from the up-down direction or an oblique direction. Meanwhile, in Fig. 2, only a part of the first index projection optical system 45 and the second index projection optical system 46 is illustrated for convenience of description. Meanwhile, the second index projection optical system 46 is also used as an anterior ocular segment illumination that illuminates the anterior ocular segment of the examinee's eye E. In addition, the second index projection optical system 46 can also be used as an index for measuring the shape of the cornea. In addition, the first index projection optical system 45 and the second index projection optical system 46 are not limited to a dot-shaped light source. For example, the systems may be a ring-shaped light source or a linear light source.

### <Observation Optical System>

For example, the observation optical system (image capture optical system) 50 shares the objective lens 14 and the dichroic mirror 29 in the subjective measurement optical system 25 and the objective measurement optical system 10, and includes an imaging lens 51 and a two-dimensional image capture element 52. For example, the image capture element 52 has an imaging surface disposed at a position substantially conjugated with the anterior ocular segment of the examinee's eye E. For example, an output from the image capture element 52 is input to the control section 70. Accordingly, an anterior ocular segment image of the examinee's eye E is captured by the two-dimensional image capture element 52 and is displayed on the monitor 4. Meanwhile, the observation optical system 50 also serves as an optical system that detects an alignment index image formed on the cornea of the examinee's eye E by the first index projection optical system 45 and the second index projection optical system 46, and the position of the alignment index image is detected by the control section 70.

### <Internal Configuration of Subjective Optometry Apparatus>

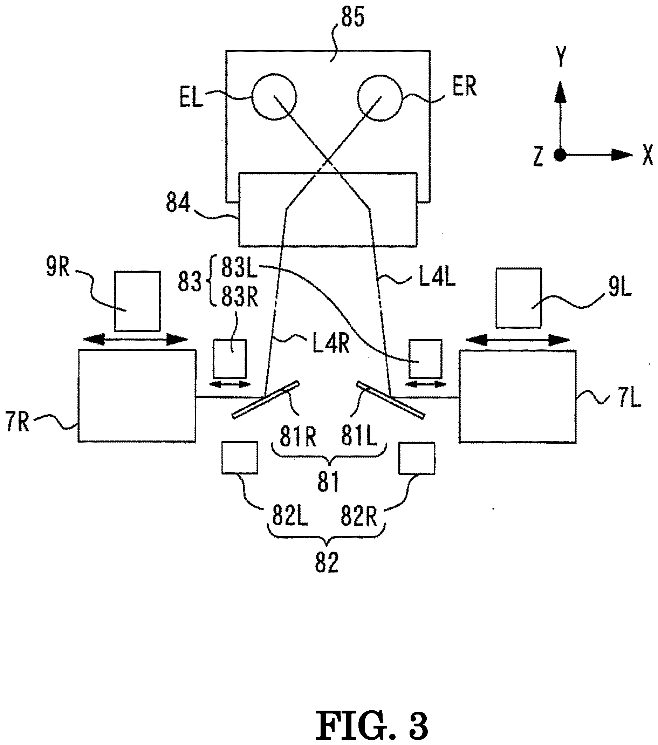

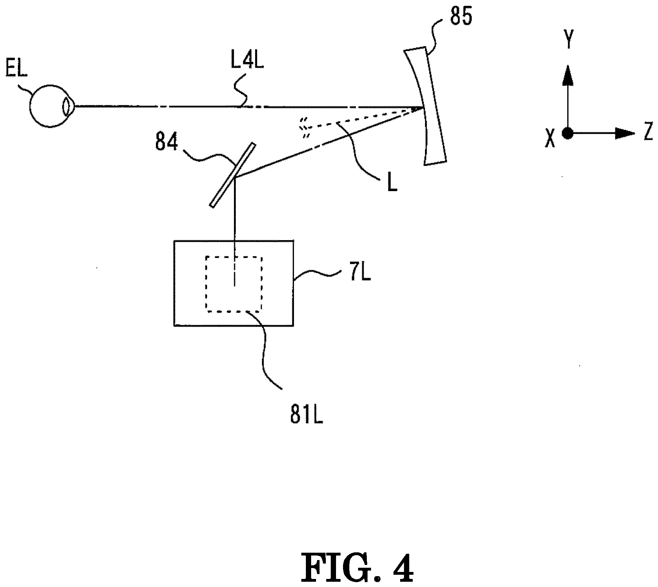

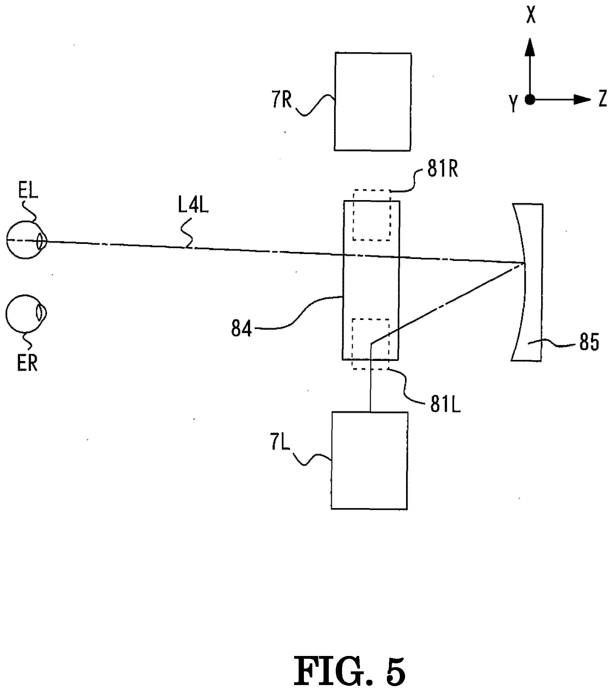

Hereinafter, the internal configuration of the subjective optometry apparatus 1 will be described. Fig. 3 is a schematic configuration view when the inside of the subjective optometry apparatus 1 according to the present example is seen from the front (a direction A of Fig. 1). Fig. 4 is a schematic configuration view when the inside of the subjective optometry apparatus 1 according to the present example is seen from the side (a direction B of Fig. 1). Fig. 5 is a schematic configuration view when the inside of the subjective optometry apparatus 1 according to the present example is seen from above (a direction C of Fig. 1). Meanwhile, in Figs. 4 and 5, only the optical axis of the left eye measurement means 7L is illustrated for convenience of description.

For example, the subjective optometry apparatus 1 includes subjective measurement means and objective measurement means. For example, in the subjective measurement means and the objective measurement means, the target light flux from the measurement means 7 passes through the optical path deviated from the optical axis L of the optical element (for example, the concave surface mirror 85 which will be described later) and is guided to the examinee's eye E. For example, the optical axis L in the present example is an axis toward the center of the sphere of the concave surface mirror 85. In other words, in the present example, the target light flux is emitted obliquely to the optical axis L of the concave surface mirror 85, and the reflected luminous flux is guided to the examinee's eye E.

For example, the subjective measurement means includes the measurement means 7, the deflection mirror 81, driving means 82, driving means 83, a reflecting mirror 84, and the concave surface mirror 85. In addition, the subjective measurement means is not limited to such a configuration. For example, the configuration without the reflecting mirror 84 may be adopted. In this case, the target light flux from the measurement means 7 may be emitted obliquely to the optical axis L of the concave surface mirror 85 after passing through the deflection mirror 81. In addition, for example, the configuration with the half mirror may be adopted. In this case, the target light flux from the measurement means 7 may be emitted obliquely to the optical axis L of the concave surface mirror 85 through the half mirror, and the reflected luminous flux may be guided to the examinee's eye E. In addition, in the present example, the concave surface mirror 85 is disposed, but a convex lens may be disposed instead of the concave surface mirror 85.

For example, the objective measurement means includes the measurement means 7, the deflection mirror 81, the reflecting mirror 84, and reflecting concave surface mirror 85. In addition, the objective measurement means is not limited to such a configuration. For example, the configuration without the reflecting mirror 84 may be adopted. In this case, the target light flux from the measurement means 7 may be emitted obliquely to the optical axis L of the concave surface mirror 85 after passing through the deflection mirror 81. In addition, for example, the configuration with the half mirror may be adopted. In this case, the target light flux from the measurement means 7 may be emitted obliquely to the optical axis L of the concave surface mirror 85 through the half mirror, and the reflected luminous flux may be guided to the examinee's eye E. In addition, in the present example, the concave surface mirror 85 is disposed, but a convex lens may be disposed instead of the concave surface mirror 85.

For example, the subjective optometry apparatus 1 includes left eye driving means 9L and right eye driving means 9R, and the left eye measurement means 7L and the right eye measurement means 7R can be moved in the X direction respectively. For example, the left eye measurement means 7L and the right eye measurement means 7R are moved, and accordingly, a distance between the deflection mirror 81 and the measurement means 7 is changed, and the presentation position of a target light flux in the Z direction is changed. Accordingly, it is possible to guide the target light flux calibrated by the calibration optical system 60 to the examinee's eye E and to adjust the measurement means 7 in the Z direction such that the image of the target light flux calibrated by the calibration optical system 60 is formed on the fundus of the examinee's eye E.

For example, the deflection mirror 81 includes a right eye deflection mirror 81R and a left eye deflection mirror 81L which are provided as a pair on the left and right sides respectively. For example, the deflection mirror 81 is disposed between the calibration optical system 60 and the examinee's eye E. In other words, the calibration optical system 60 in the present example includes the right eye calibration optical system and the left eye calibration optical system which are provided as a pair on the left and right sides respectively, the left eye deflection mirror 81L is disposed between the left eye calibration optical system and a left eye ER, and the right eye deflection mirror 81R is disposed between the right eye calibration optical system and a right eye ER. For example, it is preferable that the deflection mirror 81 is disposed at a position conjugated with the pupil.

For example, the left eye deflection mirror 81L reflects a luminous flux projected from the left eye measurement means 7L, and guides the luminous flux onto the left examinee's eye EL. In addition, for example, the left eye deflection mirror 81L reflects the reflected light reflected by the left examinee's eye EL, and guides the reflected light to the left eye measurement means 7L. For example, the right eye deflection mirror 81R reflects the luminous flux projected from the right eye measurement means 7R, and guides the luminous flux to the right examinee's eye ER. In addition, for example, the right eye deflection mirror 81R reflects the reflected light reflected by the right examinee's eye ER, and guides the reflected light to the right eye measurement means 7R. Meanwhile, in the present example, a description has been given of an example of a configuration in which the deflection mirror 81 is used as a deflection member that reflects the luminous flux projected from the measurement means 7 and guides the luminous flux to the examinee's eye E, but the invention is not limited thereto. As the deflection member, any deflection member that reflects the luminous flux projected from the measurement means 7 and guides the luminous flux to the examinee's eye E may be used. Examples of the deflection member include a prism, a lens, or the like.

For example, the driving means 82 is configured with a motor (driving section) or the like. For example, the driving means 82 includes a driving means 82L for driving the left eye deflection mirror 81L and a driving means 82R for driving the right eye deflection mirror 81R. For example, the deflection mirror 81 is rotated and moved by the driving of the driving means 82. For example, the driving means 82 rotates the deflection mirror 81 around a rotation axis in the horizontal direction (X direction) and a rotation axis in the vertical direction (Y direction). In other words, the driving means 82 rotates the deflection mirror 81 in the XY directions. Meanwhile, the rotation of the deflection mirror 81 may be performed in either the horizontal direction or the vertical direction.

For example, the driving means 83 is configured with a motor (driving section) or the like. For example, the driving means 83 includes a driving means 83L for driving the left eye deflection mirror 81L and a driving means 83R for driving the right eye deflection mirror 81R. For example, the deflection mirror 81 is moved in the X direction by the driving of the driving means 83. For example, a distance between the left eye deflection mirror 81L and the right eye deflection mirror 81R is changed by the movement of the left eye deflection mirror 81L and the right eye deflection mirror 81R, and thus it is possible to change a distance between a left eye optical path and a right eye optical path in the X direction in accordance with the pupillary distance of the examinee's eye E.

Meanwhile, for example, a plurality of deflection mirrors 81 may be provided in each of the left eye optical path and the right eye optical path. Examples of the configuration include a configuration in which two deflection mirrors are provided in each of the left eye optical path and the right eye optical path (for example, two deflection mirrors in the left eye optical path, or the like). In this case, one deflection mirror may be rotated in the X direction, and the other deflection mirror may be rotated in the Y direction. For example, the deflection mirror 81 is rotated and moved, and thus it is possible to optically correct the position of an image to be formed by deflecting an apparent luminous flux for the image of the calibration optical system 60 to be formed in front of the examinee's eye.

For example, the concave surface mirror 85 is shared by the right eye measurement means 7R and the left eye measurement means 7L. For example, the concave surface mirror 85 is shared by the right eye optical path including the right eye calibration optical system and the left eye optical path including the left eye calibration optical system. In other words, the concave surface mirror 85 is disposed at a position where the concave surface mirror passes through both the right eye optical path including the right eye calibration optical system and the left eye optical path including the left eye calibration optical system. Naturally, the concave surface mirror 85 may be configured not to be shared by the right eye optical path and the left eye optical path. In other words, a configuration may also be adopted in which the concave surface mirrors are provided in each of the right eye optical path including the right eye calibration optical system and the left eye optical path including the left eye calibration optical system. For example, the concave surface mirror 85 guides the target light flux having passed through the calibration optical system to the examinee's eye E, and forms an image of the target light flux having passed through the calibration optical system in front of the examinee's eye E. In addition, in the present embodiment, the configuration using the concave surface mirror 85 has been described as an example, but the invention is not limited thereto, and various optical elements can be used. For example, as the optical element, a lens, a planar mirror, and the like can be used.

For example, the concave surface mirror 85 also serves as the subjective measurement means and the objective measurement means. For example, the target light flux projected from the subjective measurement optical system 25 is projected onto the examinee's eye E through the concave surface mirror 85. For example, the measurement light projected from the objective measurement optical system 10 is projected onto the examinee's eye E through the concave surface mirror 85. In addition, for example, the reflected light of the measurement light projected from the objective measurement optical system 10 is guided to the light receiving optical system 10b of the objective measurement optical system 10 through the concave surface mirror 85. Meanwhile, in the present example, a configuration in which the reflected light of the measurement light from the objective measurement optical system 10 is guided to the light receiving optical system 10b of the objective measurement optical system 10 through the concave surface mirror 85 has been described as an example, but the invention is not limited thereto. For example, a configuration may also be adopted in which the reflected light of the measurement light from the objective measurement optical system 10 does not go through the concave surface mirror 85.

In more detail, for example, in the present example, an optical axis between the concave surface mirror 85 and the examinee's eye E in the subjective measurement means and an optical axis between the concave surface mirror 85 and the examinee's eye E in the objective measurement means are configured as substantially the same axis. For example, in the present example, the optical axis L2 of the subjective measurement optical system 25 and the optical axis L1 of the objective measurement optical system 10 are combined with each other by the dichroic mirror 35, and are thus configured as the same axis.

### <Optical Path of Subjective Measurement Means>

Hereinafter, the optical path of the subjective measurement means will be described. For example, the subjective measurement means reflects the target light flux having passed through the calibration optical system 60 in a direction of the examinee's eye by the concave surface mirror 85 to thereby guide the target light flux to the examinee's eye E, and forms an image of the target light flux having passed through the calibration optical system 60 in front of the examinee's eye E so as to have an optically predetermined examination distance. For example, at this time, the target light flux that has passed through the calibration optical system 60 passes through the optical path deviated from the optical axis L of the concave surface mirror 85, is incident on the concave surface mirror 85, is reflected to pass through the optical path deviated from the optical axis L of the concave surface mirror 85, and is guided to the examinee's eye E. Therefore, the visual target image seen from the examinee looks as if the visual target image is located farther than the actual distance between the examinee's eye E and the display 31. In other words, by using the concave surface mirror 85, it is possible to elongate the presenting distance of the visual target with respect to the examinee's eye E, and to present the visual target image to the examinee such that the image of the target light flux is seen at the predetermined examination distance.

A more detailed description will be given. In the following description, the left eye optical path will be described as an example, but also in the right eye optical path, the same configuration as the configuration of the left eye optical path is adopted. For example, in the left eye subjective measurement means, the target light flux projected from the display 31 of the left eye measurement means 7L is incident on the astigmatism calibration optical system 63 through the projection lens 33. The target light flux having passed through the astigmatism calibration optical system 63 is incident on the correction optical system 90 through the reflecting mirror 36, the dichroic mirror 35, the dichroic mirror 29, and the objective lens 14. The target light flux having passed through the correction optical system 90 is guided toward the left eye deflection mirror 81L from the left eye measurement means 7L. The target light flux emitted from the left eye measurement means 7L and reflected by the left eye deflection mirror 81 is reflected toward the concave surface mirror 85 by the reflecting mirror 84. For example, the target light flux emitted from the display 31 reaches the left examinee's eye EL through the optical element in this manner.

Thereby, the visual target image calibrated by the calibration optical system 60 is formed on the fundus of the left examinee's eye EL based on a spectacle wearing position (for example, a position separated from the cornea apex position at approximately 12 mm) of the left examinee's eye EL. Therefore, this is equivalent to the arrangement of the astigmatism calibration optical system 63 in front of the eyes and the adjustment of a spherical power by a calibration optical system (in the present example, driving of the driving mechanism 39) of a spherical power, and thus the examinee can collimate the visual target image in a natural state through the concave surface mirror 85. Meanwhile, in the present example, the right eye optical path also has the same configuration as that of the left eye optical path, and the visual target image calibrated by a pair of left and right calibration optical systems 60 is formed on the fundi of both examinee's eyes, based on the spectacle wearing positions (for example, positions separated from the corneas apex positions at approximately 12 mm) of both the examinee's eyes ER and EL. In this manner, the examinee responds to the examiner while looking straight at the visual target in a state of a natural sight, attempts calibration by the calibration optical system 60 until the examination visual target is seen properly, and subjectively measures the optical characteristics of the examinee's eye based on the calibration value thereof.

### <Optical Path of Objective Measurement Means>

Subsequently, the optical path of the objective measurement means will be described. In the following description, the left eye optical path will be described as an example, but also in the right eye optical path, the same configuration as the configuration of the left eye optical path is adopted. For example, in the objective measurement means for the left eye, measurement light emitted from the light source 11 of the projection optical system 10a in the objective measurement optical system 10 is incident on the correction optical system 90 through the relay lens 12 to the objective lens 14. The measurement light having passed through the correction optical system 90 is projected toward the left eye deflection mirror 81L from the left eye measurement means 7L. The measurement light emitted from the left eye measurement means 7L and reflected by the left eye deflection mirror 81 is reflected toward the concave surface mirror 85 by the reflecting mirror 84. The measurement light reflected by the concave surface mirror reaches the left examinee's eye EL through the reflecting mirror 84, thereby forming a spot-shaped point light source image on the fundus of the left examinee's eye EL. At this time, a pupil projection image (projected luminous flux on the pupil) of the hole portion of the hole mirror 13 is eccentrically rotated at high speed by the prism 15 rotating around the optical axis.

Light of the point light source image formed on the fundus of the left examinee's eye EL is reflected and scattered, and is emitted to the examinee's eye E, is condensed by the objective lens 14 through the optical path through which the measurement light is transmitted, and passes through the dichroic mirror 29, the dichroic mirror 35, the prism 15, the hole mirror 13, the relay lens 16, and the mirror 17. The reflected light having passed through the components from the dichroic mirror to the mirror 17 is condensed again on the opening of the light receiving diaphragm 18, is converted into a substantially parallel luminous flux (a case of a normal vision eye) by the collimator lens 19, is extracted as a ring-shaped luminous flux by the ring lens 20, and is received by the two-dimensional image capture element 22 as a ring image. The received ring image is analyzed, and thus it is possible to objectively measure the optical characteristics of the examinee's eye E.

### <Control Section>

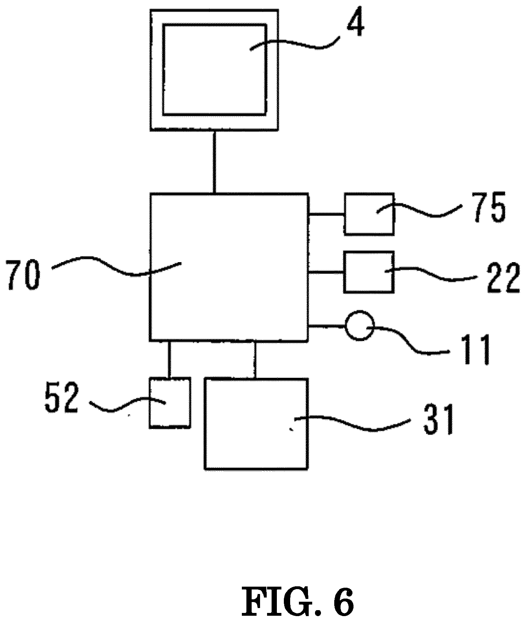

Fig. 6 is a view illustrating a control system of the subjective optometry apparatus 1 according to the present example. For example, various members, such as the monitor 4, the nonvolatile memory 75 (hereinafter, referred to as the memory 75), the measurement light source 11 included in the measurement means 7, the two-dimensional image capture element 22, the display 31, and the two-dimensional image capture element 52, are electrically connected to the control section 70. Further, for example, the driving sections which are not illustrated and are included in the driving means 9, the driving mechanism 39, the rotation mechanisms 62a and 62b, the driving means 83, and the rotation mechanisms 92a and 92b respectively, are electrically connected to the control section 70.

For example, the control section 70 includes a CPU (processor), a RAM, a ROM, and the like. For example, the CPU controls each member of the subjective optometry apparatus 1. For example, the RAM temporarily stores various pieces of information. For example, various programs for controlling the operation of the subjective optometry apparatus 1, visual target data for various examinations, an initial value, and the like are stored in the ROM. Meanwhile, the control section 70 may be configured with a plurality of control sections (that is, a plurality of processors).

For example, the memory 75 is a non-fugitive storage medium capable of holding stored contents even when the supply of power is stopped. For example, as the memory 75, a USB memory or the like which is attachably and detachably mounted to a hard disc drive, a flash ROM, and the subjective optometry apparatus 1, can be used. For example, a control program for controlling the subjective measurement means and the objective measurement means is stored in the memory 75.

### <Control Operation>

The operation of the subjective optometry apparatus 1 having the above-described configuration will be described. For example, in the present example, the objective measurement is performed with respect to the examinee's eye E by using the objective measurement optical system having the above-described configuration before starting the subjective measurement. In this case, for example, the control section 70 acquires the objectively measured refractive power, such as the spherical refractivity S, the cylinder refractivity C, the astigmatic axis angle A, the prism value Δ, and the like of the examinee's eye E. In other words, the control section 70 acquires the objective eye refractive power (objective value) of the examinee's eye E. Further, for example, the control section 70 stores the objective value in the memory 75.

For example, when starting the subjective measurement, the calibration optical system 60 is controlled based on the acquired objective eye refractive power (objective value) of the examinee's eye E. For example, the control section 70 moves the display 31 in the optical axis L2 direction based on at least one of the spherical refractivity S, the cylindrical refractivity C, the astigmatic axis angle A, the prism value Δ, and the like of the examinee's eye E which are measured objectively, and calibrates the eye refractive power of examinee's eye E.

For example, the arrangement position of the display 31 of the light projecting optical system 30 changes depending on the eye refractive power of the examinee's eye E. For example, the display 31 is disposed at a standby position at which the target light flux (that is, target light flux of 0D) which is not calibrated is not projected onto the examinee's eye E. For example, with respect to the examinee's eye E of which the eye refractive power is 0D, the standby position of the display 31 is set as the initial position of the display 31. For example, with respect to the examinee's eye E of which the eye refractive power is not 0D, the display 31 moves so as to calibrate the eye refractive power of the examinee's eye E to 0D, and the initial position of the display 31 is set to the position different from the standby position of the display 31. For example, according to this, the control section 70 can acquire the calibration power of the calibration optical system 60. In other words, the control section 70 can acquire the calibration power for calibrating the examinee's eye E to have 0D of eye refractive power, from the arrangement position of the display 31.

Further, for example, when starting the subjective measurement, a desired visual acuity value visual target (for example, a visual acuity value visual target of 1.0) is displayed on the display 31 by the control section 70. For example, when the visual acuity value visual target is presented to examinee's eye E, the examiner performs far sight measurement with respect to the examinee's eye E. For example, the visual acuity value visual target displayed on the display 31 can be switched by selecting a predetermined switch on the monitor 4. For example, the examiner performs switching to a visual target having a visual acuity value higher by one step in a case where the answer of the examinee is a correct answer. On the other hand, the examiner performs switching to a visual target having a visual acuity value lower by one step in a case where the answer of the examinee is a wrong answer. In other words, the control section 70 may switch the visual target displayed on the display 31 based on a signal for changing the visual acuity value from the monitor 4. Meanwhile, in the present example, far sight measurement will be described as an example, but the invention is not limited thereto. For example, regarding near sight measurement, measurements can be performed similar to the far sight measurement.

For example, in a state where the eye refractive power of the examinee's eye E is calibrated as described above and a desired visual acuity value visual target is displayed on the display 31, the examiner observes the presentation window 3 with the chin placed on the chin mount 5, and instructs the examinee to fixate the visual target. For example, when the anterior ocular segment of the examinee's eye E is detected by the anterior ocular segment image capture optical system 100, the control section 70 starts positioning the examinee's eye E and the measurement means 7. In other words, the control section 70 starts automatic alignment.

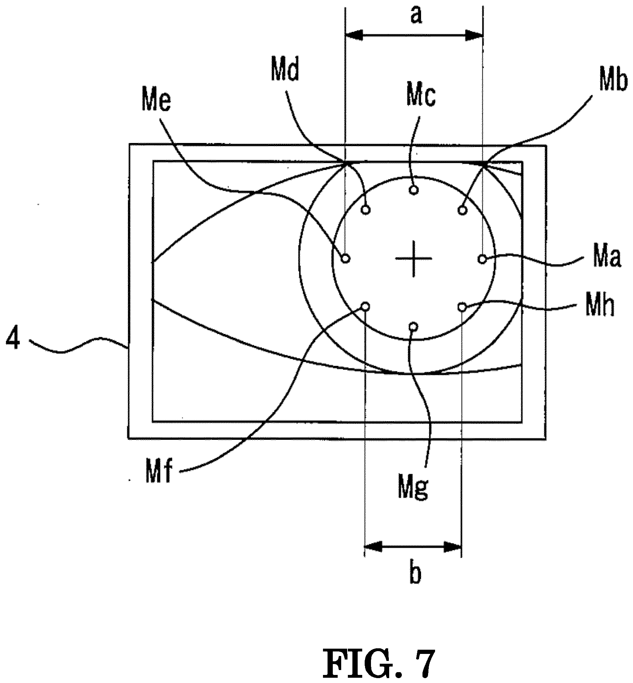

Fig. 7 is a view illustrating an anterior ocular segment image of the examinee's eye E. For example, when detecting the alignment state, the light sources of the first index projection optical system 45 and the second index projection optical system 46 are turned on. According to this, index images Ma to Mh are projected onto the examinee's eye E in a ring shape. For example, the control section 70 detects XY center coordinates (cross marks illustrated in Fig. 7) in the index images Ma to Mh as a substantial cornea apex position K. For example, the index images Ma and Me are at infinite distance and the index images Mh and Mf are at a finite distance.

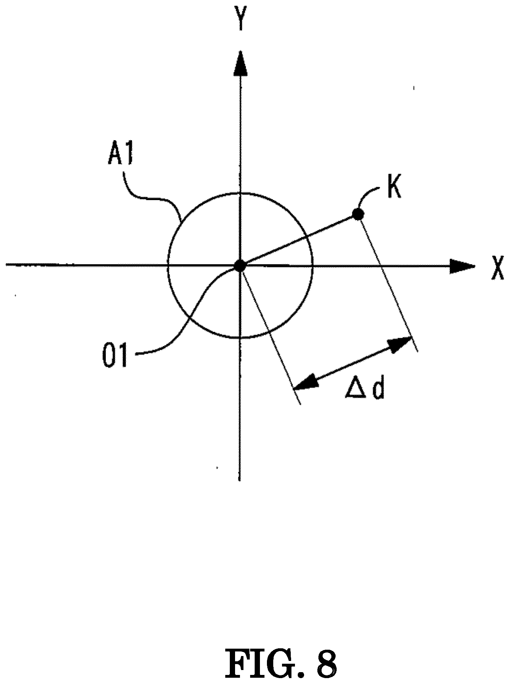

For example, the alignment state in the left-right direction (X direction) and the up-down direction (Y direction) of the examinee's eye E is determined using a preset alignment reference position O1 (refer to Fig. 8). For example, in the present example, the alignment reference position O1 is set to the cornea apex position of the examinee's eye E and to the position at which an optical axis L4L or L4R of the target light flux which is reflected by the concave surface mirror 85 toward the examinee's eye E match each other. Further, for example, a predetermined region considering the alignment reference position O1 as the center thereof is set as an alignment allowable range A1 (refer to Fig. 8) for determining whether or not the alignment is appropriate.

Fig. 8 is a view illustrating alignment control. For example, the control section 70 detects the positional shift of the target light flux in the XY directions with respect to the examinee's eye E by obtaining the deviation amount Δd of the detected substantial cornea apex position K of the examinee's eye E and the alignment reference position O1. For example, when the positional shift of the target light flux with respect to the examinee's eye E is detected, the control section 70 moves the measurement means 7 based on the detection result. For example, in the present example, by integrally moving the deflection mirror 81 and the measurement means 7 in the X direction, it is possible to perform the alignment of the examinee's eye E in the X direction (left-right direction). In addition, for example, in the present example, by integrally moving the deflection mirror 81 and the measurement means 7 in the Z direction, it is possible to perform the alignment of the examinee's eye E in the Y direction (up-down direction). In addition, the deflection mirror 81 and the measurement means 7 may not be integrated but may be configured to move separately. For example, the control section 70 adjusts the alignment in the X direction and in the Y direction such that the deviation amount Δd is within the alignment allowable range A1.

For example, when the measurement means 7 moves to the examinee's eye E as described above and the alignment is completed, the subjective measurement with respect to the examinee's eye E is started.

### <Correction of Image Plane of Image in Target light Flux>

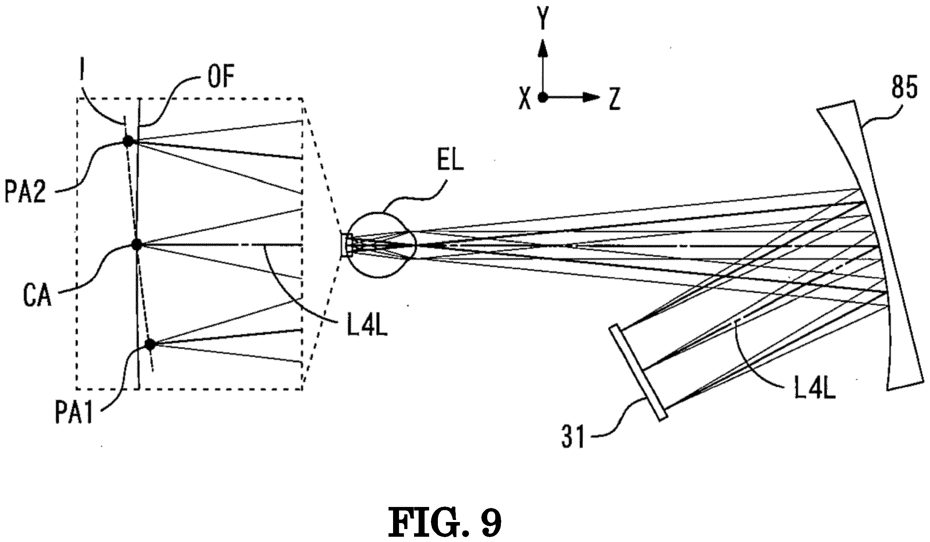

For example, in the present example, as illustrated in Figs. 2 to 5, the target light flux emitted from the display 31 passes through the optical axis L2, the optical axis L3, and the optical axis L4 (the optical axis L4L or the optical axis L4R) in order, and is guided to the examinee's eye E. At this time, the target light flux emitted from the display 31 passes through the optical path deviated from the optical axis L of the concave surface mirror 85, and is guided to the examinee's eye E. In addition, in the present example, the optical characteristics of the examinee's eye E is subjectively measured by using the target light flux guided to the examinee's eye E or the image of the target light flux guided to the examinee's eye E. For example, similar to the subjective optometry apparatus 1 in the present example, in a case where the target light flux passes through the optical path deviated from the optical axis L of the optical element (in the present example, the concave surface mirror 85), an image plane I (refer to Fig. 9) of the image of the target light flux projected onto the examinee's eye fundus is not substantially perpendicular to the optical axis, and the image plane I is inclined. In other words, as the target light flux passes through the optical path deviated from the optical axis L of the optical element, the image forming plane when the image of the target light flux is formed at a fundus OF (refer to Fig. 9) of the examinee's eye E is inclined.

Fig. 9 is a view for describing the inclination of the image plane I of the image of the target light flux. In addition, in Fig. 9, for convenience of description, only the left examinee's eye EL, the concave surface mirror 85, and the display 31 are illustrated, and the other elements are not illustrated. Further, the dotted line part in Fig. 9 is an enlarged view of a part of the left examinee's eye EL. For example, the target light flux is emitted in various directions from the display 31. In addition, for example, in the present example, as the target light flux from the center region of the display 31, the target light flux emitted from the center of the display 31 is illustrated. For example, a light condensing position CA in Fig. 9 is a light condensing position of the target light flux emitted from the center of the display 31. In addition, for example, in the present example, as the target light flux from the peripheral region of the display 31, the target light flux emitted from both ends in the up-down direction the display 31 is illustrated. For example, a light condensing position PA1 in Fig. 9 is a light condensing position of the target light flux emitted from the upper end of the display 31. In addition, for example, a light condensing position PA2 in Fig. 9 is a light condensing position of the target light flux emitted from the lower end of the display 31.

For example, the image plane I of the image of the target light flux is inclined with respect to the optical axis direction of the visual target. For example, in Fig. 9, a case where the image plane I of the image of the target light flux is inclined in the up-down direction (Y direction) with respect to the optical axis L4L is taken as an example. In addition, although not described in the present example, there is a case where the image plane I of the image of the target light flux is inclined in the left-right direction (X direction) with respect to the optical axis direction. For example, as illustrated in Fig. 9, when the light condensing position CA in the center region in the target light flux and the light condensing positions PA1 and PA2 in the peripheral region in the target light flux are shifted from the fundus OF in the left examinee's eye EL, the image plane I of the image of the target light flux guided to the left examinee's eye EL is tilted. Therefore, for example, in a case of the optical system that focuses at the center of the visual target, the visual target is in focus in the center region but the visual target of which the focus is in the peripheral region is presented to the left examinee's eye EL. In other words, in the left examinee's eye EL, the peripheral region of the visual target is blurred.

Here, for example, the control section 70 corrects the inclination of the image plane I of the image of the target light flux caused by the target light flux passing through the optical path deviated from the optical axis of the optical element. For example, in the present example, the inclination of the image plane I of the image of the image plane I of the image of the target light flux is corrected such that the image forming performance (for example, spot diagram and modulated transfer function (MTF)) of the target light flux becomes the most appropriate. For example, the inclination of the image plane I of the image of the target light flux can be corrected by changing the angle of the surface of the display 31 with respect to the optical axis. In other words, the inclination of the image plane I of the image of the target light flux can be corrected by tilting the display 31 (surface of the display 31) with respect to the optical axis.

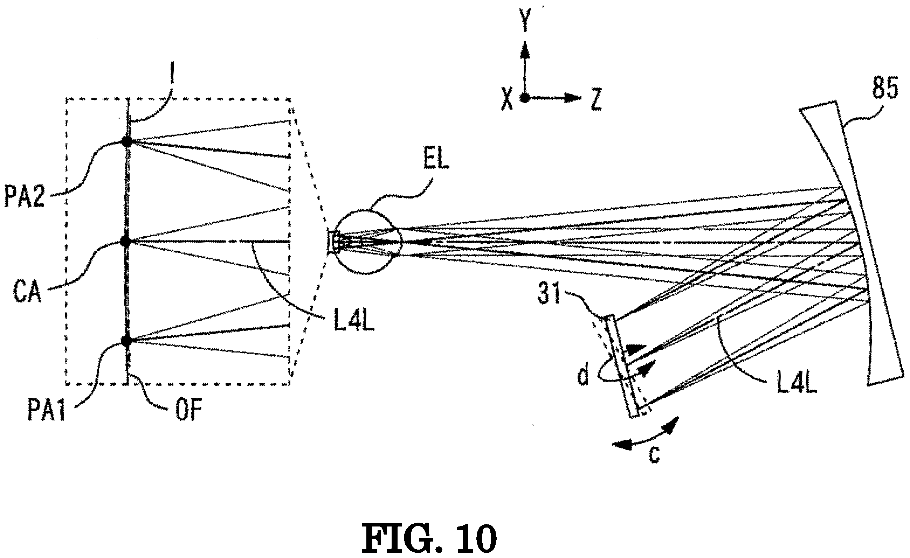

Fig. 10 is a view illustrating the image plane I of the image of the target light flux due to the tilting of the display 31. For example, in a state where the display 31 is not tilted, the light condensing position CA in the center region in the target light flux and the light condensing positions PA1 and PA2 in the peripheral region are shifted in the fundus OF of the left examinee's eye EL, and the image plane I of the image of the target light flux is inclined with respect to the optical axis L4L (refer to Fig. 9). Therefore, for example, the control section 70 in the present example rotates the display 31 in the up-down direction (the direction of the arrow c in Fig. 10) with respect to the optical axis L4L in order to correct the inclination of the image plane I. In addition, for example, the control section 70 in the present example rotates the display 31 in the left-right direction (the direction of the arrow d in Fig. 10) with respect to the optical axis L4L in order to correct the inclination of the image plane I. For example, as the control section 70 rotates the display 31 in this manner, the light condensing position CA of the center region in the target light flux and the light condensing positions PA1 and PA2 of the peripheral region are substantially perpendicular to the optical axis L4L, and the inclination of the image plane I of the image of the target light flux is corrected. In other words, the image forming plane when the image of the target light flux is formed in the fundus OF of the left examinee's eye EL is corrected substantially vertically. Therefore, the visual target focused in the center region and the peripheral region of the target light flux is presented to the left examinee's eye EL.

In addition, although the description is omitted in the present example, in the right examinee's eye ER, the image plane I of the image of target light flux is also inclined in the up-down direction (Y direction) and in the left-right direction (X direction) with respect to the optical axis L4R. For example, in this case, similar to the left examinee's eye EL, the inclination of the image plane I of the image of the target light flux can be corrected by changing the angle of the surface of the display 31 with respect to the optical axis L4R.

For example, the inclination of the image plane I of the image of the target light flux varies depending on the position at which the target light flux emitted from the display 31 is incident on the concave surface mirror 85. For example, the position at which the target light flux is incident on the concave surface mirror differs depending on the change in calibration power for calibrating the eye refractive power of examinee's eye E. In other words, when starting the subjective measurement, since the display 31 moves in accordance with the eye refractive power of the examinee's eye E, the position at which the target light flux is incident on the concave surface mirror 85 changes. Further, for example, the position at which the target light flux is incident on the concave surface mirror 85 differs depending on the alignment state between the examinee's eye E and the measurement means 7. In other words, when starting the subjective measurement, since the alignment is performed as the measurement means 7 moves with respect to the examinee's eye E, the position at which the target light flux is incident on the concave surface mirror 85 changes. Naturally, the inclination of the image plane I of the image of the target light flux is caused as long as the position when the target light flux emitted from the display 31 is incident on the concave surface mirror 85 is changed. Therefore, in a case of changing the position when the target light flux emitted from the display 31 is incident on the concave surface mirror 85, it is preferable to correct the inclination of the image plane I.

Hereinafter, a case where the image plane I of the image of the target light flux is tilted due to the change in calibration power and a case where the image plane I of the image of the target light flux is tilted in accordance with the alignment state will be described in order.

### <Correction of Image Plane based on Calibration Power>

For example, in a case where the image plane I of the image of the target light flux is tilted due to the change in calibration power, the control section 70 corrects the inclination of the image plane I of the image of the target light flux projected onto the examinee's eye fundus based on the calibration power of the calibration optical system 60. At this time, for example, the control section 70 sets the correction amount for correcting the inclination of the image plane I of the image of the target light flux based on the calibration power of the calibration optical system 60. In other words, the control section 70 sets the correction amount for correcting the inclination of the image plane I of the image of the target light flux based on the position of the display 31 moved in accordance with the eye refractive power of the examinee's eye E.

For example, in the memory 75 of the control section 70, the correction table for converting the calibration power of the calibration optical system 60 into the correction amount for correcting the inclination of the image plane I of the image of the target light flux is stored. For example, the correction table may be set in advance for each calibration power by performing experiments or simulations. For example, the control section 70 sets the correction amount that corresponds to the calibration power of the calibration optical system 60 based on the correction table. In addition, in the present example, a configuration in which the correction amount is set for each calibration power of the calibration optical system 60 in order to correct the inclination of the image plane I of the image of the target light flux is described as an example, but the invention is not limited thereto. For example, the calibration power of the calibration optical system 60 is configured to be delimited by a predetermined step (for example, 5D steps including 0D to 5D and 5D to 10D), and to set the correction amount for each step.

In addition, the control section 70 corrects the inclination of the image plane I of the image of the target light flux based on the set correction amount. For example, in the present example, the control section 70 changes the angle of the surface of the display 31 with respect to the optical axis in the up-down and left-right directions as described above, and corrects the inclination of the image plane I of the image of the target light flux tilted by the calibration power of the calibration optical system 60. According to this, the image plane I of the image of the target light flux is substantially perpendicular to the optical axis L4 (the optical axis L4L or the optical axis L4R), and in the examinee's eye E, the visual target which is focused either in the center region or in the peripheral region of the target light flux is presented.

### <Correction of Image Plane by Alignment State>

For example, in a case where the image plane I of the image of the target light flux is tilted (that is, in a case where the image plane I of the image of the target light flux is tilted depending on the alignment state) by the movement of the measurement means 7, the control section 70 corrects the inclination of the image plane I of the image of the target light flux projected onto the examinee's eye fundus based on the position information of the measurement means 7. At this time, for example, the control section 70 acquires the position information of the measurement means 7. For example, as the position information of the measurement means 7, the movement amount of the measurement means 7 may be acquired, or the position coordinates of the measurement means 7 may be acquired. In addition, for example, as the position information of the measurement means 7, the relative position information between the concave surface mirror 85 and the measurement means 7 may be acquired, or the relative position information between the examinee's eye E and the measurement means 7 may be acquired. For example, in this case, the control section 70 acquires the relative positional relationship between the concave surface mirror 85 or examinee's eye E and the measurement means 7. For example, the relative position information may be acquired by the control section 70 by detecting the position of the concave surface mirror 85 or the examinee's eye E and the position of the measurement means 7, respectively.

In addition, for example, the position information of the measurement means 7 may be configured to use the position information changed due to the adjustment of the entire position of the measurement means 7. Further, for example, the position information of the measurement means 7 may have a configuration in which the position information at which the position of at least one member (for example, a lens, or a display) of the light projecting optical system 30 of the measurement means 7 is adjusted and changed. In addition, in the description above, the configuration for acquiring the position information of the measurement means 7 has been described as an example, but the configuration for acquiring the position information of the deflection mirror 81 may be adopted. For example, in the present example, since the alignment is performed with respect to the examinee's eye E as the deflection mirror 81 and the measurement means 7 move integrally, the position information of the measurement means 7 may be acquired indirectly by using the movement amount or the position coordinates of the deflection mirror 81.

For example, the control section 70 sets the correction amount for correcting the inclination of the image plane I of the image of the target light flux based on the acquired position information of the measurement means 7. For example, in the memory 75 of the control section 70, the correction table for converting the position information of the measurement means 7 into the correction amount for correcting the inclination of the image plane I of the image of the target light flux is stored. For example, the correction table may be set in advance by performing experiments or simulations. For example, the control section 70 sets the correction amount that corresponds to the position information of the measurement means 7 based on the correction table.

In addition, for example, the control section 70 corrects the inclination of the image plane I of the image of the target light flux based on the set correction amount. For example, in the present example, the control section 70 changes the angle of the surface of the display 31 with respect to the optical axis in the up-down and left-right directions as described above, and corrects the inclination of the image plane I of the image of the target light flux tilted due to the alignment state. According to this, the image plane I of the image of the target light flux is substantially perpendicular to the optical axis L4 (the optical axis L4L or the optical axis L4R), and in the examinee's eye E, the visual target which is focused either in the center region or in the peripheral region of the target light flux is presented.

In addition, in the present example, an example in which the image plane I of the image of the target light flux is corrected respectively in a case of being tilted due to the calibration power of the calibration optical system 60 and in a case where of being inclined due to the alignment state between the examinee's eye E and the measurement means 7, is described, but the invention is not limited thereto. Naturally, in the subjective optometry apparatus 1 in the present example, a configuration for correcting the image plane I of the image in the target light flux considering both the calibration power of the calibration optical system 60 and the alignment state between the examinee's eye E and the measurement means 7 may be adopted.

### <Correction of Distortion in Target light Flux>

For example, in a case where the target light flux passes through an optical path deviated from the optical axis of the optical element (the concave surface mirror 85 in the present example), the distortion is caused in the target light flux projected onto the examinee's eye fundus. Here, in the following, the correction of the distortion in the target light flux projected onto the examinee's eye fundus will be explained.

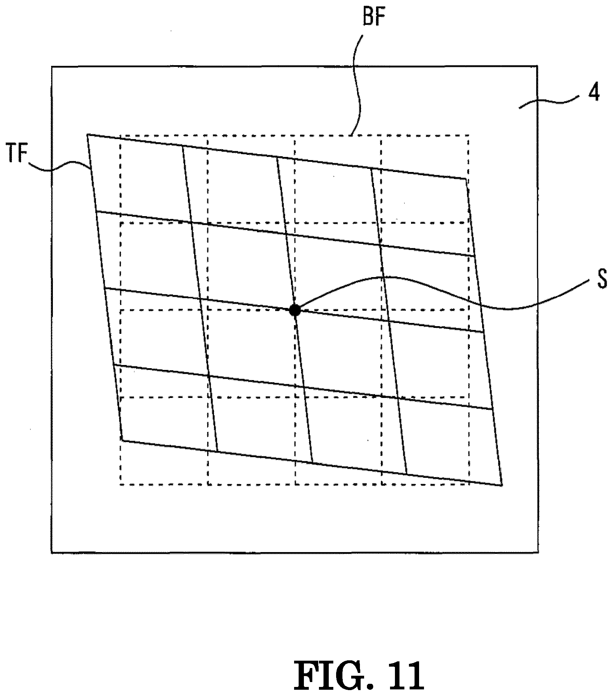

Fig. 11 is a view for describing distortion of the target light flux. In addition, in the present example, for convenience of description, the description is given assuming that a grid BF having a basic shape having the same size in the longitudinal direction and the same size in the lateral direction is displayed on the display 31 and is guided to the examinee's eye E. For example, when the distortion occurs in the target light flux, even when the grid BF having the basic shape is indicated by a dotted line is displayed on the display 31, a deformed grid TF indicated by a solid line is viewed to be projected to the examinee's eye E. In other words, for the examinee's eye E, the grid of which the size in the longitudinal direction or the size in the lateral direction is deformed is viewed to be projected due to the distortion of the target light flux. In addition, for examinee's eye E, the grid which has moved in the rotational direction around the center S of the visual target is viewed to be projected. For example, in Fig. 11, the deformed grid TF of which the size in the longitudinal direction is smaller than that of the grid BF having a basic shape, of which the size in the lateral direction is greater than that of the grid BF, and which is further rotated and moved in the counterclockwise direction around the center S of the visual target, is viewed to be displayed on the display 31. In addition, the grid (visual target) projected onto the examinee's eye E is not necessarily deformed in all of the longitudinal direction, the lateral direction, and the rotational direction, and is deformed at least in any of the lateral direction, and the rotational direction due to the position at which the target light flux emitted from the display 31 is incident on the concave surface mirror 85.

Here, for example, the control section 70 corrects the distortion of the target light flux which occurs in the target light flux passing through the optical path deviated from the optical axis of the optical element. In other words, for example, the control section 70 corrects the distortion of the target light flux projected onto the examinee's eye fundus. For example, in the present example, it is possible to correct the distortion of the target light flux projected onto the examinee's eye fundus by deforming the visual target displayed on the display 31. In other words, it is possible to perform deform the visual target and to correct the distortion of the target light flux by performing at least any one processing among the change in size in the longitudinal direction, the change in size in the lateral direction, and the movement of the visual target, in the visual target displayed on the display 31.

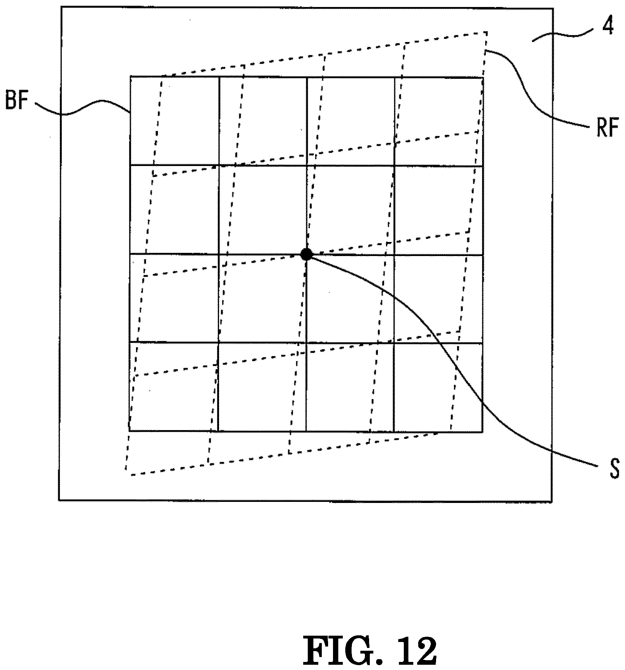

Fig. 12 is a view for describing the correction of the distortion in target light flux. In addition, in Fig. 12, the grid (visual target) displayed on the display 31 is indicated by a dotted line, and the grid (visual target) projected onto the examinee's eye E is indicated by a solid line. For example, as described with reference to Fig. 11, even when the grid BF having the basic shape is displayed on the display 31, since the target light flux is distorted, the deformed grid TF is projected onto the examinee's eye E. Therefore, for example, the control section 70 displays the visual target on the display 31 for canceling the distortion of the target light flux. For example, in the present example, the control section 70 displays a correction grid RF of which the size in the longitudinal direction is greater than that of the grid BF having a basic shape, of which the size in the lateral direction is smaller than that of the grid BF, and which has rotated and moved in the clockwise direction around the center S of the visual target, on the display 31. According to this, it is possible to correct the distortion of the target light flux guided toward the examinee's eye E. In other words, the distortion occurs in the target light flux of the correction grid RF displayed on the display 31, but the grid BF having the basic shape is projected onto the examinee's eye E.

In addition, although not described in the present example, the correction grid RF considering not only the distortion deforming in the longitudinal direction, lateral direction, and rotational direction of the visual target but also the distortion deforming into a reel type or a barrel type may be displayed on the display 31.

For example, as described above, the position at which the target light flux emitted from the display 31 is incident on the concave surface mirror 85 differs depending on the calibration power for calibrating the eye refractive power of examinee's eye E. In addition, for example, as described above, the position at which the target light flux emitted from the display 31 is incident on the concave surface mirror 85 differs depending on the alignment state between the examinee's eye E and the measurement means 7. Hereinafter, a case where the target light flux is distorted due to the change in calibration power and a case where the target light flux is distorted in accordance with the alignment state will be described in order.

### <Correction of Distortion based on Calibration Power>

For example, in a case where the target light flux is distorted due to the change in calibration power, the control section 70 corrects the distortion of the target light flux projected onto the examinee's eye fundus based on the calibration power of the calibration optical system 60. At this time, for example, the control section 70 sets the correction amount for correcting the distortion of the target light flux based on the calibration power of the calibration optical system 60. In other words, the control section 70 sets the correction amount for correcting the distortion of the target light flux based on the position of the display 31 moved in accordance with the eye refractive power of the examinee's eye E.

For example, in the memory 75 of the control section 70, the correction table for converting the calibration power of the calibration optical system 60 into the correction amount for correcting the distortion of the target light flux is stored. For example, the correction table may be set in advance by performing experiments or simulations. For example, the control section 70 sets the correction amount that corresponds to the calibration power of the calibration optical system 60 based on the correction table.

In addition, the control section 70 corrects the distortion of the target light flux based on the set correction amount. For example, in the present example, the control section 70 corrects the distortion of the target light flux by performing at least any one processing among the change in size in the longitudinal direction, the change in size in the lateral direction, and the movement of the visual target, in the visual target as described above. Accordingly, on the display 31, the correction grid RF for canceling the distortion of the target light flux which changes in accordance with the calibration power of the calibration optical system 60 is displayed, and the grid BF having the basic shape is projected onto the examinee's eye E.

### <Correction of Distortion due to Alignment State>

For example, in a case where the target light flux is distorted due to the alignment state (that is, in a case where the target light flux is distorted due to the movement of the measurement means 7 with respect to the examinee's eye E), the control section 70 corrects the distortion of target light flux projected onto the examinee's eye fundus based on the position information of the measurement means 7. For example, the alignment state is determined using the above-described index images Ma to Mh (refer to Fig. 7), and the position of the measurement means 7 with respect to the examinee's eye E is adjusted. For example, the control section 70 acquires the position information of the measurement means 7 at this time. In addition, similar to a case of correcting the image plane I of the image of the target light flux, the position information of the measurement means 7 may use the movement amount or the position coordinates of the measurement means 7, or may use the relative position information between the examinee's eye E and the measurement means 7. Further, the position information of the measurement means 7 may be obtained indirectly by obtaining the position information of the deflection mirror 81.

For example, the control section 70 sets the correction amount for correcting the distortion of the target light flux based on the position information when acquiring the position information of the measurement means 7. For example, in the memory 75 of the control section 70, the correction table for converting the position information of the measurement means 7 into the correction amount for correcting the distortion of the target light flux is stored. For example, the correction table may be set in advance by performing experiments or simulations. For example, the control section 70 sets the correction amount that corresponds to the position information of the measurement means 7 based on the correction table.

In addition, for example, the control section 70 corrects the distortion of the target light flux based on the set correction amount. For example, in the present example, the control section 70 corrects the distortion of the target light flux by performing at least any one processing among the change in size in the longitudinal direction, the change in size in the lateral direction, and the movement of the visual target, in the visual target as described above. Accordingly, on the display 31, the correction grid RF for canceling the distortion of the target light flux which changes in accordance with the alignment state is displayed, and the grid BF having the basic shape is projected onto the examinee's eye E.

In addition, in the present example, a case of correcting the distortion of the target light flux which occurs in each of the change in calibration power of the calibration optical system 60 and the change in alignment state between the examinee's eye E and the measurement means 7, is described as an example, but the invention is not limited thereto. Naturally, in the subjective optometry apparatus 1 in the present example, a configuration for correcting the distortion of the target light flux considering both the calibration power of the calibration optical system 60 and the alignment state between the examinee's eye E and the measurement means 7 may be adopted.

As described above, for example, the subjective optometry apparatus according to the present example includes the correction means which corrects the inclination of the image plane of the image of the target light flux caused by the target light flux passing through the optical path deviated from the optical axis of the optical element. According to this, it is possible to subjectively measure the optical characteristics of the examinee's eye in a state where the inclination of the image plane of the image of the target light flux is reduced. Therefore, an examiner can perform subjective measurement with respect to examinee's eye with high accuracy.

In addition, for example, the subjective optometry apparatus according to the present example includes the correction means which corrects the distortion of the target light flux caused by the target light flux passing through the optical path deviated from the optical axis of the optical element. According to this, it is possible to subjectively measure the optical characteristics of the examinee's eye in a state where the distortion of the target light flux is reduced. Therefore, an examiner can perform subjective measurement with respect to examinee's eye with high accuracy.

In addition, for example, the subjective optometry apparatus in the present example corrects the inclination of the image plane of the image of the target light flux based on the calibration power of the calibration optical system. Accordingly, it is possible to suppress the inclination of the image plane of the image of the target light flux caused by the change in calibration power of the calibration optical system in accordance with the eye refractive power of the examinee's eye. Therefore, an examiner can perform subjective measurement with respect to examinee's eye with high accuracy.

In addition, for example, the subjective optometry apparatus in the present example corrects the distortion of the target light flux based on the calibration power of the calibration optical system. According to this, it is possible to suppress the distortion of the target light flux caused by the change in calibration power of the calibration optical system in accordance with the eye refractive power of the examinee's eye. Therefore, an examiner can perform subjective measurement with respect to examinee's eye with high accuracy.

Further, for example, the subjective optometry apparatus according to the present example includes the shift detection means for detecting the positional shift of the target light flux, the moving means for moving the measurement unit based on the detection result of the shift detection means; and position information acquisition means for acquiring the position information of the measurement unit. According to this, it is possible to suppress the inclination of the image plane of the image of the target light flux caused when performing the alignment of the examinee's eye and the measurement means. In addition, according to this, it is possible to suppress the distortion of the target light flux caused when performing the alignment of the examinee's eye and the measurement means. Therefore, an examiner can perform subjective measurement with respect to examinee's eye with high accuracy.

Further, for example, the subjective optometry apparatus in the present example changes the angle of the display surface relative to the optical axis based on the set correction amount. According to this, the examiner can easily correct the inclination of the image plane of the image of the target light flux.

Further, for example, the subjective optometry apparatus in the present example deforms the visual target displayed on the display based on the set correction amount. According to this, the examiner can easily correct the distortion of the target light flux.

Further, for example, in the subjective optometry apparatus according to the present example, by using the optical element, the target light flux or the image of the target light flux can be guided to the examinee's eye so as to have an optically predetermined examination distance. Therefore, it is possible to reduce the size of the apparatus without requiring an extra member or space for guiding the image of the target light flux to the examinee's eye so as to have an actual distance.

### <Modification Example>

In addition, there is a case where the size of the visual target displayed on the display 31 appears to be slightly different between a case where the examinee's eye E sees the display 31 from the front direction and a case where the examinee's eye E sees the display 31 from the oblique direction. In other words, there is a case where the visual target in a case where the examinee's eye E sees the display 31 from the oblique direction is seen to be longer longitudinally and laterally compared to a case where the examinee's eye E sees the display 31 from the front direction. For example, in the present example, the display 31 is disposed to be orthogonal to the optical axis, and the examinee's eye E can see the display 31 from the front direction. However, for example, when correcting the inclination of the image plane I of the image in the target light flux, in order to change the angle of the surface of the display 31 with respect to the optical axis, there is a case where the examinee's eye E sees the visual target displayed on the display 31 from the oblique direction and the size of the visual target changes.

Therefore, for example, the control section 70 may be configured to correct the change in size of the visual target as described above by adjusting the size of the visual target displayed on the display 31. For example, in this case, the control section 70 may acquire the correction amount for correcting the change in size of the visual target based on the angle at which the display 31 is tilted. Further, for example, the control section 70 may adjust the size of the visual target displayed on the display 31 based on the acquired correction amount. According to this, even when the display 31 is tilted for correcting the inclination of the image plane I of the image in the target light flux, it is possible to present the visual target having the same size as that in a case where the display 31 is viewed from the front direction by the examinee's eye E.

In addition, in the present example, a configuration in which the control section 70 acquires the correction amount that corresponds to the calibration power of the calibration optical system 60 by using the correction table is described as an example, but the invention is not limited thereto. For example, the control section 70 may be configured to acquire the correction amount that corresponds to the calibration power of the calibration optical system 60 based on the arithmetic expression. In this case, for example, the arithmetic expression for performing arithmetic operation processing is set by performing experiments and simulations in advance, and is stored in the memory 75 of the control section 70. For example, the control section 70 may be configured to acquire the correction amount that corresponds to the calibration power using the arithmetic expression and to perform the arithmetic operation processing for correcting the image plane I of the image of the target light flux. In addition, for example, the control section 70 may be configured to acquire the correction amount that corresponds to the calibration power using the arithmetic expression and to perform the arithmetic operation processing for correcting the distortion of the target light flux.

Similarly, in the present example, a configuration in which the control section 70 acquires the correction amount that corresponds to the position information of the measurement means 7 by using the correction table is described as an example, but a configuration may be adopted in which the correction amount that corresponds to the position information of the measurement means 7 is acquired from the arithmetic expression. In this case, for example, the control section 70 may perform the arithmetic operation processing for correcting the image plane I of the image of the target light flux by using the arithmetic expression. In addition, for example, the control section 70 may perform the arithmetic operation processing for correcting the distortion of the target light flux by using the arithmetic expression.

In addition, in the present example, a configuration in which the inclination of the image plane I of the image of the target light flux is corrected based on the calibration power of the calibration optical system 60 or in accordance with the alignment state of the examinee's eye E is described as an example, but the invention is not limited thereto. For example, in the present example, the inclination of the image plane I of the image of the target light flux may be corrected based on the pupillary distance of the examinee's eye E. For example, the pupillary distance of the examinee's eye E is measured during the objective measurement and is stored in the memory 75 of the control section 70.

For example, at the start of the subjective measurement, the control section 70 calls the pupillary distance of examinee's eye E from the memory 75 and sets the pupillary distance. In addition, the pupillary distance of the examinee's eye E may be configured to be set by examiner inputting the pupillary distance on the monitor 4, or may be configured to receive and set the pupillary distance acquired by another apparatus, and a predetermined pupillary distance (for example, average pupillary distance of a person) may be configured to be automatically set.

For example, the control section 70 integrally moves the deflection mirror 81 and the measurement means 7 in the X direction and rotates the deflection mirror 81 in accordance with the set pupillary distance of the examinee's eye E. For example, according to this, the left eye deflection mirror 81L and the right eye deflection mirror 81R respectively move, and the distance between the left eye deflection mirror 81L and the right eye deflection mirror 81R is changed. Therefore, the distance in the X direction between the left eye optical path and the right eye optical path can be changed in accordance with the pupillary distance of examinee's eye E.

For example, since the measurement means 7 also moves in accordance with the pupillary distance of the examinee's eye E, the position at which the target light flux is incident on the concave surface mirror 85 changes, and the image plane I of the image of the target light flux is tilted. Here, for example, similar to the correction of the image plane I by the alignment state, the control section 70 may tilt the display 31 and correct inclination of the display 31 and inclination of the image plane I of the image of the target light flux based on the position information (for example, the movement amount or the position coordinates of the measurement means 7, the relative position information between the examinee's eye E and the measurement means 7, and the like) of the measurement means 7.

In addition, above, the inclination of the image plane I of the image of the target light flux is corrected based on the pupillary distance of the examinee's eye E, but when the measurement means 7 moves in accordance with the pupillary distance of the examinee's eye E, the position at which the target light flux is incident on the concave surface mirror 85 changes and the distortion occurs in the target light flux. Therefore, for example, similar to the correction of the distortion due to the alignment state, the control section 70 may correct the distortion of the target light flux by performing at least any one processing among the change in size in the longitudinal direction of the visual target, the change in size in the lateral direction, and the movement of the visual target, based on the convergence amount of the light projecting optical system 30.

In addition, in the present example, a configuration in which the inclination of the image plane I of the image of the target light flux is corrected based on the calibration power of the calibration optical system 60 or in accordance with the alignment state of the examinee's eye E is described as an example, but the invention is not limited thereto. For example, in the present example, the inclination of the image plane I of the image of the target light flux may be corrected based on a convergence amount of the examinee's eye E.

For example, in the present example, the inclination of the image plane I of the image of the target light flux may be corrected based on a convergence angle of the examinee's eye E. For example, the control section 70 may control the light projecting optical system 30 and change the convergence angle by the deflection mirror 81. In other words, the control section 70 may change the convergence amount (convergence angle) of the light projecting optical system 30 by rotating the deflection mirror 81.

For example, since the deflection mirror 81 rotates as described above when the convergence amount is changed, the position at which the target light flux is incident on the concave surface mirror 85 changes, and the image plane I of the image of the target light flux is tilted. Here, for example, the control section 70 may correct the inclination of the image plane I of the image of the target light flux based on the convergence amount of the light projecting optical system 30. For example, in the present embodiment, the control section 70 uses both the rotation amount of the deflection mirror 81 (for example, the rotation angle, the position information, and the position coordinates) and the position information of the measurement means 7 to set the correction amount for correcting the inclination of the image plane I of the image of the target light flux. For example, the control section 70 acquires and sets the correction amount that corresponds to the rotation amount of the deflection mirror 81 and the position information of the measurement means 7 from the memory 75. In addition, for example, the memory 75 may store the correction table for acquiring the correction amount, or may store the arithmetic expression. According to this, the rotation amount of the deflection mirror 81 and the position information of the measurement means 7 are converted into the correction amounts. In addition, for example, the control section 70 corrects the inclination of the image plane I of the image of the target light flux based on the set correction amount. For example, in the present example, as described above, the control section 70 can correct the inclination I of the image plane of the image of the target light flux by changing the angle of the surface of the display 31 based on the correction amount.

In addition, above, the inclination of the image plane I of the image of the target light flux is corrected based on the convergence amount of the light projecting optical system 30, but when the deflection mirror 81 rotates in accordance with the change in convergence amount, the position at which the target light flux is incident on the concave surface mirror 85 changes and the distortion occurs in the target light flux. Therefore, for example, the control section 70 sets the correction amount for correcting the distortion of the target light flux using the rotation amount of the deflection mirror 81 and the position information of the measurement means 7. For example, the correction table or the arithmetic expression for acquiring the correction amount may be stored in the memory 75, and the rotation amount of the deflection mirror 81 and the position information of the measurement means 7 may be converted into the correction amount. For example, the control section 70 can correct the distortion of the target light flux by performing at least any one processing among the change in size in the longitudinal direction of the visual target, the change in size in the lateral direction, and the movement of the visual target based on the set correction amount.

In addition, in the present example, the alignment state of the measurement means 7 in the left-right direction (X direction) and in the up-down direction (Y direction) of the examinee's eye E is described, but the alignment state of the measurement means 7 in the front-back direction (Z direction) of the examinee's eye E may be taken into consideration. For example, in a state where the measurement means 7 is aligned in the Z direction of the examinee's eye E, an image interval a from the index image Ma to Me at a finite distance illustrated in Fig. 7 and an image interval b from the index image Mh to Mf at an infinite distance have a certain constant ratio. For example, in a state where the measurement means 7 is not aligned in the Z direction of the examinee's eye E, the image interval from the index image Ma to Me at a finite distance rarely changes, and the image interval from the index image Mh to Mf at an in finite distance changes. For example, the control section 70 compares the image ratio (that is, a/b) of the image interval a of the index images Ma and Me at a finite distance and the image interval b of the index images Mh and Mf at an infinite distance, it is possible to detect the positional shift in the Z direction of the target light flux with respect to the examinee's eye E. In addition, for the details of the above-described structure, refer to JP-A-6-46999.

For example, the control section 70 may detect the positional shift of the target light flux with respect to the examinee's eye E in the X direction, in the Y direction, and in the Z direction, may move the measurement means 7 based on the detection, and may adjust the alignment of the examinee's eye E and the measurement means 7. For example, in the present example, by integrally moving the deflection mirror 81 and the measurement means 7 in the optical axis L4 direction, it is possible to perform the alignment of the examinee's eye E in the Z direction (front-back direction).

For example, in a case where the measurement means 7 moves in the X direction, in the Y direction, and in the Z direction, since the position of the target light flux incident on the concave surface mirror 85 changes, the inclination occurs in the image plane I of the image of the target light flux guided to the examinee's eye E. Therefore, for example, the control section 70 may be configured to correct the image plane I of the image of the target light flux by using the position information in the X direction, in the Y direction, and in the Z direction of the measurement means 7 with respect to the examinee's eye E. Similarly, for example, in a case where the measurement means 7 moves in the X direction, in the Y direction, and in the Z direction, since the position of the target light flux incident on the concave surface mirror 85 changes, the distortion occurs in the target light flux guided to the examinee's eye E. Therefore, for example, the control section 70 may be configured to correct the distortion of the target light flux by using the position information in the X direction, in the Y direction, and in the Z direction of the measurement means 7 with respect to the examinee's eye E.

In addition, in the present example, a configuration in which the alignment in the X direction, in the Y direction, and in the Z direction is adjusted by integrally driving the deflection mirror 81 and the measurement means 7 is described, but the invention is not limited thereto. For example, in the present example, any configuration may be adopted as long as the positional relationship of the examinee's eye E, the subjective measurement means, and the objective measurement means can be adjusted by driving the deflection mirror 81 and the measurement means 7. In other words, any configuration may be adopted as long as the adjustment in the X direction, in the Y direction, and in the Z direction is possible such that the target light flux from the light projecting optical system 30 is formed on the fundus of the examinee's eye E. For example, in this case, a configuration may also be adopted in which the subjective optometry apparatus 1 is moved by providing a configuration in which the subjective optometry apparatus 1 can be moved in the XYZ directions with respect to the chin mount 5. Further, for example, the deflection mirror 81 may be fixed and only the measurement means 7 may be moved. Further, for example, a configuration may be adopted in which the adjustment in the X direction, in the Y direction, and in the Z direction is possible only by the deflection mirror 81. In this case, for example, a configuration can be adopted in which the deflection mirror 81 is rotationally driven in the Z direction and the distance between the deflection mirror 81 and the measurement means 7 is changed.

In addition, in the present example, a configuration in which the eye refractive power of examinee's eye E is acquired by the objective measurement optical system of the subjective optometry apparatus 1 is described, but the present invention is not limited thereto. For example, a configuration may be adopted in which the eye refractive power of the examinee's eye E is acquired by the subjective measurement optical system of the subjective optometry apparatus 1. In this case, the calibration power of the calibration optical system 60 can be acquired using the objective eye refractive power (objective value) as described in the present example, and can be acquired using the subjective eye refractive power (subjective value) acquired in the subjective measurement. For example, a configuration may be adopted in which the subjective value acquired during the subjective measurement is stored in the memory 75 at any time and the control section 70 calls the subjective value in accordance with the alignment state between the examinee's eye E and the measurement means 7.

In addition, in the present example, a configuration in which the eye refractive power of examinee's eye E is acquired by the objective measurement optical system of the subjective optometry apparatus 1 is described, but the present invention is not limited thereto. For example, the eye refractive power of the examinee's eye E may use the objective value or the subjective value of the object eye E acquired by another apparatus. For example, in this case, a configuration can be adopted in which the subjective optometry apparatus 1 has a reception function for receiving the eye refractive power from another apparatus. In addition, for example, in this case, a configuration may be adopted in which the examiner inputs the eye refractive power of the examinee's eye E.

In addition, in the present example, a configuration in which the alignment between the examinee's eye E and the measurement means 7 is performed before the start of the subjective measurement is described as an example, but the invention is not limited thereto. For example, a configuration may be adopted in which the alignment between the examinee's eye E and the measurement means 7 is performed even during the subjective measurement. For example, in this case, the control section 70 may always detect the alignment shift between the examinee's eye E and the measurement means 7 even after the alignment of the measurement means 7 with respect to the examinee's eye E is completed, and may perform tracking control (tracking) for constantly detecting the movement of the examinee's eye E in the XYZ directions. For example, in subjective optometry apparatus 1 having the configuration, the control section 70 can constantly correct the inclination of the image plane I of the image of the target light flux projected onto the examinee's eye along with the movement of the examinee's eye E. In addition, in the subjective optometry apparatus 1 having the configuration, the control section 70 can constantly correct the distortion of the target light flux projected onto the examinee's eye along with the movement of the examinee's eye E.

In addition, in the present example, a configuration in which the inclination of the image plane I of the image of the target light flux is corrected by changing the angle of the surface of the display 31 with respect to the optical axis is described as an example, but the invention is not limited thereto. For example, in the present example, the inclination of the image plane I of the image of the target light flux may be corrected by moving the optical element based on the set correction amount. For example, in this case, the optical element of the light projecting optical system 30 may be used, or an optical element may be separately provided.

For example, in a case of correcting the inclination of the image plane I of the image of the target light flux by using the optical element of the light projecting optical system 30, based on the correction amount set by the control section 70, the optical element may be tilted in the optical axis direction of the light projecting optical system 30. For example, in the present example, the projection lens 33, the projection lens 34, the objective lens 14, and the like can be tilted as optical elements. In addition, a configuration may be adopted in which either one of the projection lens and the objective lens is tilted, or a configuration may be adopted in which the plurality of lenses are combined and tilted.

Further, for example, in a case of correcting the inclination of the image plane I of the image of the target light flux by separately providing the optical element, the optical element may be inserted into and removed from the optical axis of the light projecting optical system 30 based on the set correction amount. For example, the optical element may be inserted and removed anywhere on the optical axis through which the target light flux guided from the display 31 to the examinee's eye E passes. In other words, the optical element may be inserted or removed anywhere on the optical axis L2 and on the optical axis L3. For example, as the optical element, a lens (a convex lens, a concave lens), a prism, a mirror, or the like can be used.

In this case, for example, the subjective optometry apparatus in the present example includes the moving optical element which is movable with respect to the optical path of the light projecting optical system, and the driving means for moving the moving optical element with respect to the optical path of the light projecting optical system. Further, for example, in the subjective optometry apparatus in the present example, it is possible to move the moving optical element based on the correction amount. Therefore, the examiner can dispose the optical element at an appropriate position with respect to the examinee's eye and can correct the inclination of the image plane of the image of the target light flux with high accuracy.

In addition, in the present example, a configuration in which the distortion of the target light flux is corrected by deforming the visual target displayed on the display 31 in advance is described as an example, but the invention is not limited thereto. For example, in the present example, similar to a case of correcting the inclination of the image plane I of the image of the target light flux, the distortion of the target light flux may be corrected by moving the optical element based on the set correction amount. For example, in this case, the optical element of the light projecting optical system 30 may also be used, or an optical element may be separately provided.

For example, in a case of correcting the distortion of the target light flux by using the optical element of the light projecting optical system 30, a configuration may be adopted in which any of the projection lens 33, the projection lens 34, the objective lens 14, and the like is tilted, or a configuration may be adopted in which the plurality of lenses are combined and tilted. Further, for example, in a case of correcting the distortion of the target light flux by providing an optical element separately, the lens (the convex lens, the concave lens), the prism, the mirror, and the like are inserted and removed on the optical axis through which the target light flux passes. For example, the distortion of the target light flux may be corrected as the control section 70 tilts or inserts and removes the optical element based on the correction amount set in this manner.

In this case, for example, the subjective optometry apparatus in the present example includes the moving optical element which is movable with respect to the optical path of the light projecting optical system, and the driving means for moving the moving optical element with respect to the optical path of the light projecting optical system. Further, for example, in the subjective optometry apparatus in the present example, it is possible to move the moving optical element based on the correction amount. Therefore, the examiner can dispose the optical element at an appropriate position with respect to the examinee's eye and can correct the distortion of the target light flux with high accuracy.

In addition, in the present example, based on the calibration power of the calibration optical system 60, a configuration in which the distortion caused by the target light flux passing through the optical path deviated from the optical axis L of the optical element (the concave surface mirror 85 in the present example) is corrected is described as an example, but the invention is not limited thereto. For example, when the target light flux passes through the optical path deviated from the optical axis L of the optical element, various aberrations such as astigmatism, spherical aberration, coma aberration, chromatic aberration, distortion aberration and the like are generated. For example, the control section 70 in the present example may be configured to correct the above-described aberration based on the calibration power of the calibration optical system 60.

## Claims

1. A subjective optometry apparatus (1) for subjectively measuring optical characteristics of an examinee's eye, comprising:
a light projecting optical system (30) that projects a target light flux to the examinee's eye;
an optical element (85) that guides the target light flux; and
a correction portion that corrects distortion of the target light flux caused by the target light flux passing through an optical path deviated from an optical axis of the optical element (85),
wherein the target light flux passes through the optical path deviated from the optical axis of the optical element (85) to be guided to the examinee's eye, and
the subjective optometry apparatus (1) subjectively measures the optical characteristics of the examinee's eye using the target light flux guided to the examinee's eye,
**characterized in that** the apparatus further comprises a calibration optical system (60) that is disposed in an optical path from the light projecting optical system (30) to the examinee's eye, and changes optical characteristics of the target light flux; and
the optical element (85) guides the target light flux of which the optical characteristics is changed by the calibration optical system to the examinee's eye.

2. The subjective optometry apparatus (1) according to claim 1,
wherein the correction portion corrects the distortion of the target light flux based on a calibration power of the calibration optical system (60).

3. The subjective optometry apparatus (1) according to claim 1 or 2, further comprising:
a measurement unit (7) that accommodates the light projecting optical system (30); and
a shift detection portion (70) that detects a positional shift of the target light flux with respect to the examinee's eye,
wherein the correction portion corrects the distortion of the target light flux based on a detection result detected by the shift detection portion (70).

4. The subjective optometry apparatus (1) according to any one of claims 1 to 3,
wherein the light projecting optical system (30) includes a display (31), and the target light flux is emitted as a visual target is displayed on the display (31), and
the correction portion deforms the visual target displayed on the display (31) to correct the distortion of the target light flux.

5. The subjective optometry apparatus (1) according to claim 4,
wherein the correction portion deforms the visual target to correct the distortion of the target light flux by performing at least one processing among a change in size in a longitudinal direction, a change in size in a lateral direction, and a movement of the visual target, in the visual target displayed on the display.

6. The subjective optometry apparatus (1) according to any one of claims 1 to 3, further comprising:
a moving optical element that is movable with respect to an optical path of the light projecting optical system (30); and
a driving portion that moves the moving optical element with respect to the optical path of the light projecting optical system (30),
wherein the correction portion that controls the driving portion for moving the moving optical element to correct the distortion of the target light flux.

7. The subjective optometry apparatus (1) according to any one of claims 1 to 5,
wherein the optical element (85) guides the target light flux to the examinee's eye so as to have an optically predetermined examination distance.

8. The subjective optometry apparatus (1) according to claim 1, **characterized in that** the correction portion is adapted to correct aberration caused by the target light flux passing through an optical path deviated from an optical axis of the optical element (85), based on a calibration power of the calibration optical system (60).

9. The subjective optometry apparatus (1) according to claim 1 or 8,
wherein the correction portion corrects inclination of an image plane of an image of the target light flux caused by the target light flux passing through the optical path deviated from the optical axis of the optical element (85).

10. The subjective optometry apparatus (1) according to claim 9,
wherein the correction portion corrects the inclination of the image plane of the image of the target light flux based on a calibration power of the calibration optical system (60).

11. The subjective optometry apparatus (1) according to claim 9 or 10, further comprising:
a measurement unit that accommodates the light projecting optical system (30); and
a shift detection portion that detects a positional shift of the target light flux with respect to the examinee's eye,
wherein the correction portion corrects the inclination of the image plane of the image of the target light flux based on a detection result detected by the shift detection portion.

12. The subjective optometry apparatus (1) according to any one of claims 9 to 11,
wherein the light projecting optical system (30) includes a display, and the target light flux is emitted as a visual target is displayed on the display, and
the correction portion changes an angle of a surface of the display with respect to the optical axis to correct the inclination of the image plane of the image of the target light flux.

13. The subjective optometry apparatus (1) according to any one of claims 9 to 11, further comprising:
a moving optical element that is movable with respect to an optical path of the light projecting optical system (30); and
a driving portion that moves the moving optical element with respect to the optical path of the light projecting optical system (30),
wherein the correction portion controls the driving portion for moving the moving optical element to correct the inclination of the image plane of the image of the target light flux.

14. The subjective optometry apparatus (1) according to any one of claims 9 to 13,
wherein the optical element (85) guides an image of the target light flux to the examinee's eye so as to have an optically predetermined examination distance.

15. A subjective optometry program used in a subjective optometry apparatus (1) including a light projecting optical system (30) that projects a target light flux to an examinee's eye, a calibration optical system (60) that is disposed in an optical path from the light projecting optical system (30) to the examinee's eye, and changes optical characteristics of the target light flux, and an optical element (85) that guides the target light flux of which the optical characteristics is changed by the calibration optical system (60) to the examinee's eye, in which the target light flux passes through the optical path deviated from an optical axis of the optical element (85) to be guided to the examinee's eye, and optical characteristics of the examinee's eye are subjectively measured using the target light flux guided to the examinee's eye,
the subjective optometry program, when executed by a processor of the subjective optometry apparatus, causing the subjective optometry apparatus to perform a correction step of correcting distortion of the target light flux caused by the target light flux passing through an optical path deviated from an optical axis of the optical element (85).

16. A subjective optometry program used in a subjective optometry apparatus (1) including a light projecting optical system (30) that projects a target light flux to examinee's eye, a calibration optical system (60) that is disposed in an optical path from the light projecting optical system (30) to the examinee's eye, and changes optical characteristics of the target light flux, and an optical element (85) that guides the target light flux of which the optical characteristics is changed by the calibration optical system (60) to the examinee's eye, in which the target light flux passes through the optical path deviated from an optical axis of the optical element (85) to be guided to the examinee's eye, and optical characteristics of the examinee's eye are subjectively measured using the target light flux guided to the examinee's eye,
the subjective optometry program, when executed by a processor of the subjective optometry apparatus, causing the subjective optometry apparatus to perform a correction step of correcting inclination of an image plane of an image of the target light flux caused by the target light flux passing through an optical path deviated from an optical axis of the optical element (85).

## Patentansprüche

1. Subjektive optometrische Vorrichtung (1) zum subjektiven Messen optischer Eigenschaften des Auges einer Testperson, umfassend:
ein optisches Lichtprojektionssystem (30), das einen Ziel-Lichtstrom auf das Auge der Testperson projiziert;
ein optisches Element (85), das den Ziel-Lichtstrom leitet; und
einen Korrekturabschnitt, der eine Verzerrung des Ziel-Lichtstroms korrigiert, die dadurch verursacht wird, dass der Ziel-Lichtstrom durch einen von einer optischen Achse des optischen Elements (85) abweichenden Strahlengang verläuft,
wobei der Ziel-Lichtstrom durch den von der optischen Achse des optischen Elements (85) abweichenden Strahlengang verläuft, um zum Auge der Testperson geleitet zu werden, und
die subjektive optometrische Vorrichtung (1) die optischen Eigenschaften des Auges der Testperson subjektiv unter Verwendung des zum Auge der Testperson geleiteten Ziel-Lichtstroms misst,
**dadurch gekennzeichnet, dass** die Vorrichtung ferner ein optisches Kalibrierungssystem (60) umfasst, das in einem Strahlengang vom optischen Lichtprojektionssystem (30) zum Auge der Testperson angeordnet ist und optische Eigenschaften des Ziel-Lichtstroms ändert; und
das optische Element (85) den Ziel-Lichtstrom, dessen optische Eigenschaften durch das optische Kalibrierungssystem geändert wurden, zum Auge der Testperson leitet.

2. Subjektive optometrische Vorrichtung (1) nach Anspruch 1,
wobei der Korrekturabschnitt die Verzerrung des Ziel-Lichtstroms basierend auf einer Kalibrierungsleistung des optischen Kalibrierungssystems (60) korrigiert.

3. Subjektive optometrische Vorrichtung (1) nach Anspruch 1 oder 2, ferner umfassend:
eine Messeinheit (7), in der das optische Lichtprojektionssystem (30) untergebracht ist; und
einen Verschiebungserfassungsabschnitt (70), der eine Positionsverschiebung des Ziel-Lichtstroms in Bezug auf das Auge der Testperson erfasst,
wobei der Korrekturabschnitt die Verzerrung des Ziel-Lichtstroms basierend auf einem Erfassungsergebnis korrigiert, das vom Verschiebungserfassungsabschnitt (70) erfasst wird.

4. Subjektive optometrische Vorrichtung (1) nach einem der Ansprüche 1 bis 3,
wobei das optische Lichtprojektionssystem (30) eine Anzeige (31) umfasst und der Ziel-Lichtstrom emittiert wird, während ein visuelles Ziel auf der Anzeige (31) angezeigt wird, und
der Korrekturabschnitt das auf der Anzeige (31) angezeigte visuelle Ziel verformt, um die Verzerrung des Ziel-Lichtstroms zu korrigieren.

5. Subjektive optometrische Vorrichtung (1) nach Anspruch 4,
wobei der Korrekturabschnitt das visuelle Ziel verformt, um die Verzerrung des Ziel-Lichtstroms dadurch zu korrigieren, dass zumindest eine Verarbeitung einer Größenänderung in Längsrichtung und/oder einer Größenänderung in Querrichtung und/oder einer Bewegung des visuellen Ziels in dem auf der Anzeige angezeigten visuellen Ziels durchgeführt wird.

6. Subjektive optometrische Vorrichtung (1) nach einem der Ansprüche 1 bis 3, ferner umfassend:
ein bewegliches optisches Element, das in Bezug auf einen Strahlengang des optischen Lichtprojektionssystems (30) beweglich ist; und
einen Antriebsabschnitt, der das bewegliche optische Element in Bezug auf den Strahlengang des optischen Lichtprojektionssystems (30) bewegt,
wobei der Korrekturabschnitt, den Antriebsabschnitt zum Bewegen des beweglichen optischen Elements steuert, um die Verzerrung des Ziel-Lichtstroms zu korrigieren.

7. Subjektive optometrische Vorrichtung (1) nach einem der Ansprüche 1 bis 5,
wobei das optische Element (85) den Ziel-Lichtstrom zum Auge der Testperson leitet, sodass ein optisch vorgegebener Untersuchungsabstand entsteht.

8. Subjektive optometrische Vorrichtung (1) nach Anspruch 1, **dadurch gekennzeichnet, dass** der Korrekturabschnitt zum Korrigieren einer Abweichung, die durch den Ziel-Lichtstrom verursacht wird, der durch einen von einer optischen Achse des optischen Elements (85) abweichenden Strahlengang verläuft, basierend auf einer Kalibrierungsleistung des optischen Kalibrierungssystems (60) eingerichtet ist.

9. Subjektive optometrische Vorrichtung (1) nach Anspruch 1 oder 8,
wobei der Korrekturabschnitt die Neigung einer Bildebene eines Bildes des Ziel-Lichtstroms korrigiert, die durch den Ziel-Lichtstrom verursacht wird, der durch den von der optischen Achse des optischen Elements (85) abweichenden Strahlengang verläuft,

10. Subjektive optometrische Vorrichtung (1) nach Anspruch 9,
wobei der Korrekturabschnitt die Neigung der Bildebene des Bildes des Ziel-Lichtstroms basierend auf einer Kalibrierungsleistung des optischen Kalibrierungssystems (60) korrigiert.

11. Subjektive optometrische Vorrichtung (1) nach Anspruch 9 oder 10, ferner umfassend:
eine Messeinheit, in der das optische Lichtprojektionssystem (30) untergebracht ist; und
einen Verschiebungserfassungsabschnitt, der eine Positionsverschiebung des Ziel-Lichtstroms in Bezug auf das Auge der Testperson erfasst,
wobei der Korrekturabschnitt die Neigung der Bildebene des Bildes des Ziel-Lichtstroms basierend auf einem Erfassungsergebnis korrigiert, das vom Verschiebungserfassungsabschnitt erfasst wird.

12. Subjektive optometrische Vorrichtung (1) nach einem der Ansprüche 9 bis 11,
wobei das optische Lichtprojektionssystem (30) eine Anzeige umfasst und der Ziel-Lichtstrom emittiert wird, wenn ein visuelles Ziel auf der Anzeige angezeigt wird, und
der Korrekturabschnitt einen Winkel einer Oberfläche der Anzeige in Bezug auf die optische Achse ändert, um die Neigung der Bildebene des Bildes des Ziel-Lichtstroms zu korrigieren.

13. Subjektive optometrische Vorrichtung (1) nach einem der Ansprüche 9 bis 11, ferner umfassend:
ein sich bewegendes optisches Element, das in Bezug auf einen Strahlengang des optischen Lichtprojektionssystems (30) beweglich ist; und
einen Antriebsabschnitt, der das bewegliche optische Element in Bezug auf den Strahlengang des optischen Lichtprojektionssystems (30) bewegt,
wobei der Korrekturabschnitt den Antriebsabschnitt zum Bewegen des sich bewegenden optischen Elements steuert, um die Neigung der Bildebene des Bildes des Ziel-Lichtstroms zu korrigieren.

14. Subjektive optometrische Vorrichtung (1) nach einem der Ansprüche 9 bis 13,
wobei das optische Element (85) ein Bild des Ziel-Lichtstroms zum Auge der Testperson führt, sodass ein optisch vorbestimmter Untersuchungsabstand entsteht.

15. Subjektives optometrisches Programm, das in einer subjektiven optometrischen Vorrichtung (1) verwendet wird, die ein optisches Lichtprojektionssystem (30), das einen Ziel-Lichtstrom auf das Auge einer Testperson projiziert, ein optisches Kalibrierungssystem (60), das in einem Strahlengang vom optischen Lichtprojektionssystem (30) zum Auge der Testperson angeordnet ist und die optischen Eigenschaften des Ziel-Lichtstroms ändert, und ein optisches Element (85) umfasst, das den Ziel-Lichtstrom, dessen optische Eigenschaften durch das optische Kalibrierungssystem (60) geändert wurden, zum Auge der Testperson leitet, wobei der Ziel-Lichtstrom durch den von einer optischen Achse des optischen Elements (85) abweichenden Strahlengang verläuft, um zum Auge der Testperson geleitet zu werden, und optische Eigenschaften des Auges der Testperson subjektiv unter Verwendung des zum Auge der Testperson geleiteten Ziel-Lichtstroms gemessen werden,
wobei das subjektive optometrische Programm beim Ausführen durch einen Prozessor der subjektiven optometrischen Vorrichtung bewirkt, dass die subjektive optometrische Vorrichtung einen Korrekturschritt zum Korrigieren einer Verzerrung des Ziel-Lichtstroms durchführt, die dadurch verursacht wird, dass der Ziel-Lichtstrom durch einen von einer optischen Achse des optischen Elements (85) abweichenden Strahlengang verläuft.

16. Subjektives optometrisches Programm, das in einer subjektiven optometrischen Vorrichtung (1) verwendet wird, die ein optisches Lichtprojektionssystem (30), das einen Ziel-Lichtstrom auf das Auge einer Testperson projiziert, ein optisches Kalibrierungssystem (60), das in einem Strahlengang vom optischen Lichtprojektionssystem (30) zum Auge der Tastperson angeordnet ist und die optischen Eigenschaften des Ziel-Lichtstroms ändert, und ein optisches Element (85) umfasst, das den Ziel-Lichtstrom, dessen optische Eigenschaften durch das optische Kalibrierungssystem (60) geändert wurden, zum Auge der Testperson leitet, wobei der Ziel-Lichtstrom durch den von einer optischen Achse des optischen Elements (85) abweichenden Strahlengang verläuft, um zum Auge der Testperson geleitet zu werden, und optische Eigenschaften des Auges der Testperson subjektiv unter Verwendung des zum Auge der Testperson geleiteten Ziel-Lichtstroms gemessen werden,
wobei das subjektive optometrische Programm beim Ausführen durch einen Prozessor der subjektiven optometrischen Vorrichtung bewirkt, dass die subjektive optometrische Vorrichtung einen Korrekturschritt zum Korrigieren einer Neigung einer Bildebene eines Bildes des Ziel-Lichtstroms durchführt, der durch den Ziel-Lichtstrom verursacht wird, der durch einen von einer optischen Achse des optischen Elements (85) abweichenden Strahlengang verläuft.

## Revendications

1. Appareil d'optométrie subjective (1) pour mesurer subjectivement des caractéristiques optiques de l'œil d'une personne examinée, comprenant :
un système optique de projection de lumière (30) qui projette un flux lumineux cible sur l'œil de la personne examinée ;
un élément optique (85) qui guide le flux lumineux cible ; et
une partie de correction qui corrige la distorsion du flux lumineux cible provoquée par le flux lumineux cible traversant un trajet optique dévié d'un axe optique de l'élément optique (85), où le flux lumineux cible traverse le trajet optique dévié de l'axe optique de l'élément optique (85) pour être guidé vers l'œil de la personne examinée, et
l'appareil d'optométrie subjective (1) mesure subjectivement les caractéristiques optiques de l'œil de la personne examinée en utilisant le flux lumineux cible guidé vers l'œil de la personne examinée,
**caractérisé en ce que** l'appareil comprend en outre un système optique d'étalonnage (60) qui est disposé dans un trajet optique du système optique de projection de lumière (30) à l'œil de la personne examinée, et modifie les caractéristiques optiques du flux lumineux cible ; et
l'élément optique (85) guide le flux lumineux cible dont les caractéristiques optiques sont modifiées par le système optique d'étalonnage vers l'œil de la personne examinée.

2. Appareil d'optométrie subjective (1) selon la revendication 1,
dans lequel la partie de correction corrige la distorsion du flux lumineux cible sur la base d'une puissance d'étalonnage du système optique d'étalonnage (60).

3. Appareil d'optométrie subjective (1) selon la revendication 1 ou 2, comprenant en outre :
une unité de mesure (7) qui reçoit le système optique de projection de lumière (30) ; et
une partie de détection de décalage (70) qui détecte un décalage de position du flux lumineux cible par rapport à l'œil de la personne examinée,
dans lequel la partie de correction corrige la distorsion du flux lumineux cible sur la base d'un résultat de détection détecté par la partie de détection de décalage (70).

4. Appareil d'optométrie subjective (1) selon l'une quelconque des revendications 1 à 3,
dans lequel le système optique de projection de lumière (30) comprend un dispositif d'affichage (31), et le flux lumineux cible est émis à mesure qu'une cible visuelle est affichée sur le dispositif d'affichage (31), et
la partie de correction déforme la cible visuelle affichée sur le dispositif d'affichage (31) pour corriger la distorsion du flux lumineux cible.

5. Appareil d'optométrie subjective (1) selon la revendication 4,
dans lequel la partie de correction déforme la cible visuelle pour corriger la distorsion du flux lumineux cible en effectuant au moins un traitement parmi un changement de taille dans une direction longitudinale, un changement de taille dans une direction latérale et un mouvement de la cible visuelle, dans la cible visuelle affichée sur le dispositif d'affichage.

6. Appareil d'optométrie subjective (1) selon l'une quelconque des revendications 1 à 3, comprenant en outre :
un élément optique mobile qui est mobile par rapport à un trajet optique du système optique de projection de lumière (30) ; et
une partie d'entraînement qui déplace l'élément optique mobile par rapport au trajet optique du système optique de projection de lumière (30),
dans lequel la partie de correction commande la partie d'entraînement pour déplacer l'élément optique mobile afin de corriger la distorsion du flux lumineux cible.

7. Appareil d'optométrie subjective (1) selon l'une quelconque des revendications 1 à 5,
dans lequel l'élément optique (85) guide le flux lumineux cible vers l'œil de la personne examinée de manière à avoir une distance d'examen optiquement prédéterminée.

8. Appareil d'optométrie subjective (1) selon la revendication 1, **caractérisé en ce que** la partie de correction est adaptée pour corriger l'aberration provoquée par le flux lumineux cible traversant un trajet optique dévié d'un axe optique de l'élément optique (85), sur la base d'une puissance d'étalonnage du système optique d'étalonnage (60).

9. Appareil d'optométrie subjective (1) selon la revendication 1 ou 8, dans lequel la partie de correction corrige l'inclinaison d'un plan d'image d'une image du flux lumineux cible provoquée par le flux lumineux cible traversant le trajet optique dévié de l'axe optique de l'élément optique (85).

10. Appareil d'optométrie subjective (1) selon la revendication 9,
dans lequel la partie de correction corrige l'inclinaison du plan d'image de l'image du flux lumineux cible sur la base d'une puissance d'étalonnage du système optique d'étalonnage (60).

11. Appareil d'optométrie subjective (1) selon la revendication 9 ou 10, comprenant en outre :
une unité de mesure qui reçoit le système optique de projection de lumière (30) ; et
une partie de détection de décalage qui détecte un décalage de position du flux lumineux cible par rapport à l'œil de la personne examinée,
dans lequel la partie de correction corrige l'inclinaison du plan d'image de l'image du flux lumineux cible sur la base d'un résultat de détection détecté par la partie de détection de décalage.

12. Appareil d'optométrie subjective (1) selon l'une quelconque des revendications 9 à 11,
dans lequel le système optique de projection de lumière (30) comprend un dispositif d'affichage, et le flux lumineux cible est émis à mesure qu'une cible visuelle est affichée sur le dispositif d'affichage, et
la partie de correction modifie un angle d'une surface du dispositif d'affichage par rapport à l'axe optique pour corriger l'inclinaison du plan d'image de l'image du flux lumineux cible.

13. Appareil d'optométrie subjective (1) selon l'une quelconque des revendications 9 à 11, comprenant en outre :
un élément optique mobile qui est mobile par rapport à un trajet optique du système optique de projection de lumière (30) ; et
une partie d'entraînement qui déplace l'élément optique mobile par rapport au trajet optique du système optique de projection de lumière (30),
dans lequel la partie de correction commande la partie d'entraînement pour déplacer l'élément optique mobile afin de corriger l'inclinaison du plan d'image de l'image du flux lumineux cible.

14. Appareil d'optométrie subjective (1) selon l'une quelconque des revendications 9 à 13,
dans lequel l'élément optique (85) guide une image du flux lumineux cible vers l'œil de la personne examinée de manière à avoir une distance d'examen optiquement prédéterminée.

15. Programme d'optométrie subjective utilisé dans un appareil d'optométrie subjective (1) comprenant un système optique de projection de lumière (30) qui projette un flux lumineux cible sur l'œil d'une personne examinée, un système optique d'étalonnage (60) qui est disposé dans un trajet optique du système optique de projection de lumière (30) à l'œil de la personne examinée, et modifie les caractéristiques optiques du flux lumineux cible, et un élément optique (85) qui guide le flux lumineux cible dont les caractéristiques optiques sont modifiées par le système optique d'étalonnage (60) vers l'œil de la personne examinée, où le flux lumineux cible traverse le trajet optique dévié d'un axe optique de l'élément optique (85) pour être guidé vers l'œil de la personne examinée, et les caractéristiques optiques de l'œil de la personne examinée sont mesurées subjectivement en utilisant le flux lumineux cible guidé vers l'œil de la personne examinée,
le programme d'optométrie subjective, lorsqu'il est exécuté par un processeur de l'appareil d'optométrie subjective, amenant l'appareil d'optométrie subjective à effectuer une étape de correction consistant à corriger la distorsion du flux lumineux cible provoquée par le flux lumineux cible traversant un trajet optique dévié d'un axe optique de l'élément optique (85).

16. Programme d'optométrie subjective utilisé dans un appareil d'optométrie subjective (1) comprenant un système optique de projection de lumière (30) qui projette un flux lumineux cible sur l'œil de la personne examinée, un système optique d'étalonnage (60) qui est disposé dans un trajet optique du système optique de projection de lumière (30) à l'œil de la personne examinée, et modifie les caractéristiques optiques du flux lumineux cible, et un élément optique (85) qui guide le flux lumineux cible dont les caractéristiques optiques sont modifiées par le système optique d'étalonnage (60) vers l'œil de la personne examinée, où le flux lumineux cible traverse le trajet optique dévié d'un axe optique de l'élément optique (85) pour être guidé vers l'œil de la personne examinée, et les caractéristiques optiques de l'œil de la personne examinée sont mesurées subjectivement en utilisant le flux lumineux cible guidé vers l'œil de la personne examinée,
le programme d'optométrie subjective, lorsqu'il est exécuté par un processeur de l'appareil d'optométrie subjective, amenant l'appareil d'optométrie subjective à effectuer une étape de correction consistant à corriger l'inclinaison d'un plan d'image d'une image du flux lumineux cible provoquée par le flux lumineux cible traversant un trajet optique dévié d'un axe optique de l'élément optique (85).
